# EUROPEAN PATENT SPECIFICATION

(11) **EP 3 250 182 B1**
(45) Date of publication and mention of the grant of the patent: **26.04.2023**
(21) Application number: 16701516.3
(22) Date of filing: 26.01.2016
(51) Int. Cl.: A61K 9/00, A61K 9/20, A61K 45/06, A61K 31/47, A61P 31/06, A61P 43/00

(54) **DISPERSIBLE COMPOSITIONS**
DISPERGIERBARE ZUSAMMENSETZUNGEN
COMPOSITIONS DISPERSIBLES

(30) Priority: 27.01.2015 IN 264MU2015
(43) Date of publication of application: 06.12.2017
(73) Proprietor: Janssen Pharmaceutica NV, 2340 Beerse (BE)
(72) Inventor: GUPTA, Manish, Kumar, Mumbai 400080 Maharashtra (IN); MARATHE, Shripad, Wasudeo, Mumbai 40080 Maharashtra (IN); TAMBWEKAR, Kaustubh, Ramesh, Mumbai 40080 Maharashtra (IN); NAIR, Shreedevi, Velayudhan, Mumbai 400101 (IN)
(74) Representative: Purewal, Savroop
(86) International application number: PCT/EP2016/051545
(87) International publication number: WO 2016/120258

(56) References cited:
- WO-A1-2008/068231
- WO-A1-2010/026526
- Anonymous: "A Study to Assess the Relative Bioavailability of TMC207 Following Single-Dose Administrations of Two Pediatric Formulations in Healthy Adult Participants", ClinicalTrials.gov , March 2014 (2014-03), XP002754986, Retrieved from the Internet: URL:https://www.clinicaltrials.gov/ct2/sho w/record/NCT01803373?term=TMC207
- Erica Lessem ET AL: "An Activist's Guide to BEDAQUILINE (Sirturo)", , 1 February 2013 (2013-02-01), pages 1-8, XP55251839, Retrieved from the Internet: URL:http://www.treatmentactiongroup.org/si tes/g/files/g450272/f/201303/Bedaquiline.p df [retrieved on 2016-02-19]
- Kalavathy D J ET AL: "Preparation and Evaluation of Dispersible Tablets of A Model Antibiotic Drug", Int. J. Pharm. Sci. Rev. Res. Jan - Feb International Journal of Pharmaceutical Sciences Review and Research, 1 January 2013 (2013-01-01), pages 21-29, XP55252144, Retrieved from the Internet: URL:http://globalresearchonline.net/journa lcontents/v18-1/05.pdf [retrieved on 2016-02-22]
- Kalavathy D J ET AL: "Preparation and Evaluation of Dispersible Tablets of A Model Antibiotic Drug", Int. J. Pharm. Sci. Rev. Res. Jan - Feb International Journal of Pharmaceutical Sciences Review and Research, 1 January 2013 (2013-01-01), pages 21-29, XP055252144, Retrieved from the Internet: URL:http://globalresearchonline.net/journa lcontents/v18-1/05.pdf [retrieved on 2016-02-22]
- Stuart L Cantor ET AL: "Chapter 8: Pharmaceutical Granulation Processes, Mechanism, and the Use of Binders" In: "Pharmaceutical Dosage Forms : Tablets - Unit Operations and Mechanical Properties", 3 June 2008 (2008-06-03), CRC Press LLC, XP055601290, pages 261-302,

## Description

The present invention concerns a pharmaceutical composition containing a certain antibacterial product, bedaquiline fumarate, as active ingredient. More specifically, the invention relates to a dispersible, or disintegrating, tablet, a process for preparing it, as well as its use in the treatment of antibacterial diseases such as tuberculosis. Such novel compositions are particularly suited to the paediatric population. It can also suit the geriatric population.

The active ingredient in this case is bedaquiline in the form of a fumarate salt: (alpha S, beta R)-6-bromo-alpha-[2-(dimethylamino)ethyl]-2-methoxy-alpha-1-naphthalenyl-beta -phenyl-3-quinolineethanol, in particular (alpha S, beta R)-6-bromo-alpha-[2-(dimethylamino)ethyl] -2-methoxy-alpha-1 -naphthalenyl-beta-phenyl-3 - quinolineethanol (2E)-2-butenedioate (1:1) and may be represented by the following formula:

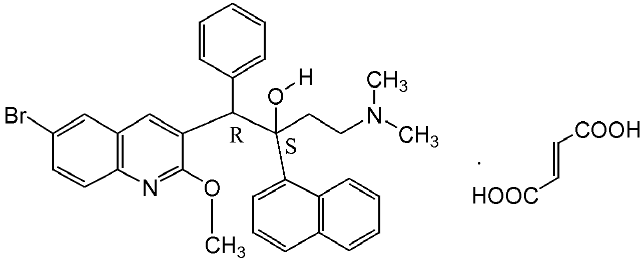

The fumarate salt of the present invention can be prepared by reacting the corresponding free base with fumaric acid in the presence of a suitable solvent, such as for example isopropanol.

This product Sirturo^{™} containing the active ingredient has already received marketing approval in some territories including the US, Russia, the EU, South Africa and the Republic of Korea.

The utility of the invention arises from the active ingredient, and salt thereof, being known to show activity against *Mycobacteria* including drug resistant strains, in particular *Mycobacterium tuberculosis, M. bovis, M. avium, M. leprae and M. marinum,* especially against *Mycobacterium tuberculosis,* including drug-resistant *M. tuberculosis* strains. The active ingredient, including salt thereof, shows activity against active, sensitive, susceptible *Mycobacteria* strains and latent, dormant, persistent *Mycobacteria* strains.

International patent application WO 2004/011436 first disclosed the activity of the free base of bedaquiline against *Mycobacteria.* Later documents such as international patent applications WO 2005/117875 and WO 2006/067048 disclose the further uses in the treatment of *inter alia* drug resistant tuberculosis and latent tuberculosis. International patent application WO 2008/068231 first described the suitablility of the fumarate salt as a drug product indicating its acceptable bioavilaibility. The fumarate salt of bedaquiline is described as non-hygroscopic and stable. This document also discloses the preparation of certain formulations and tablets containing bedaquiline fumarate.

In particular, WO 2008/068231 discloses the preparation of a drug formulation comprising bedaquiline fumarate salt, where a powder mixture is obtained and compressed into tablets. Such a formulation does not have adequate dispersibility/disintegrating properties.

*Mycobacterium tuberculosis* results in more than 2 million deaths per year and is the leading cause of mortality in people infected with HIV. In spite of decades of tuberculosis (TB) control programs, about 2 billion people are infected by *M. tuberculosis,* though asymptomatically. About 10% of these individuals are at risk of developing active TB during their lifespan. There is thus a high need for drugs to treat active TB.

The global epidemic of TB is fuelled by infection of HIV patients with TB and rise of multi-drug resistant TB strains (MDR-TB). The reactivation of latent TB is a high risk factor for disease development and accounts for 32% deaths in HIV infected individuals. To control TB epidemic, the need is to discover new drugs that can also kill dormant or latent bacilli. The dormant TB can get reactivated to cause disease by several factors like suppression of host immunity by use of immunosuppressive agents like antibodies against tumor necrosis factor α or interferon-y. In case of HIV positive patients the only prophylactic treatment available for latent TB is two- three months regimens of rifampicin, pyrazinamide. The efficacy of the treatment regime is still not clear and furthermore the length of the treatments is an important constrain in resource-limited environments. Hence there is a drastic need to identify new drugs, which can act as chemoprophylatic agents for individuals harboring latent TB bacilli.

The tubercle bacilli enter healthy individuals by inhalation; they are phagocytosed by the alveolar macrophages of the lungs. This leads to potent immune response and formation of granulomas, which consist of macrophages infected with *M. tuberculosis* surrounded by T cells. After a period of 6-8 weeks the host immune response cause death of infected cells by necrosis and accumulation of caseous material with certain extracellular bacilli, surrounded by macrophages, epitheloid cells and layers of lymphoid tissue at the periphery. In case of healthy individuals, most of the mycobacteria are killed in these environments but a small proportion of bacilli still survive and are thought to exist in a non-replicating, hypometabolic state and are tolerant to killing by anti-TB drugs like isoniazid. These bacilli can remain in the altered physiological environments even for individual's lifetime without showing any clinical symptoms of disease. However, in 10% of the cases these latent bacilli may reactivate to cause disease. One of the hypothesis about development of these persistent bacteria is patho-physiological environment in human lesions namely, reduced oxygen tension, nutrient limitation, and acidic pH. These factors have been postulated to render these bacteria phenotypically tolerant to major anti-mycobacterial drugs.

Although pharmaceutical formulations of bedaquiline fumarate have been developed for the adult population, to date there has been no paediatric formulation developed and pursued. There are many approaches to developing drug formulations including direct compression, dry granulation and wet granulation. There remains individual challenges to each approach and it is also dependent on the drug that is to be formulated too. Further, for the paediatric population, it may be desired to have a dispersible tablet, and this may also provide additional challenges. This is particularly so from the perspective of devising such tablets that have a suitably fast dispersion time.

Among the techniques used to prepare tablets, direct compression is the simplest, involving only blending and compression. This has the advantage of speed of production as it requires fewer unit operations, less machinery and is, as a consequence, more efficient.

Direct compression is followed by other manufacturing processes, such as dry or wet granulation.

There is now provided a dispersible composition (e.g. tablet) comprising bedaquiline fumarate as the active ingredient as described in the claims. This may be referred to herein as "the (dispersible) tablet of the invention".

By "dispersible", we mean a composition (e.g. tablet) that disintegrates in appropriate media, for example aqueous media (water) or other suitable media or vehicles for administration (e.g. milk, juice or even semi-solid like vehicles such as yogurt, apple sauce). More particularly, we mean that the tablet disintegrates in a low volume of water such that it disperses (for example evenly and/or rapidly) by mild swirling. For example a 100 mg tablet composition may be evenly dispersed within 90 seconds in about 50 ml of water. Hence, an equivalent of 1 mg of tablet composition weight in 0.5 ml water may be evenly dispersed within 90 seconds. More preferably, an equivalent of 1 mg of tablet composition weight in 0.5 ml water may be evenly dispersed within 60 secs, more preferably within 45 seconds. In particular it may be dispersed in around (e.g. within) 30 seconds. Such dispersion times/ratios are applicable in particular for composition weights between 20 mg and 400 mg (particularly between about 50 mg and about 200 mg) as shown by the examples hereinafter. Hence, in particular a tablet composition of 100 mg may be evenly dispersed in 50 ml water around (or within) 30 seconds. Such a dispersion may pass through a sieve screen with a nominal mesh aperture of 710 µm.

However, even though an equivalent of 1 mg of composition (e.g. tablet composition) weight in 0.5 ml water is referred to in the context of dispersion, it is the intrinsic properties of the tablet formulation that are key. For example, dispersion may occur in a much smaller volume of fluid, for example a 100 mg composition may also be dispersed in a much lower quantity of water, for example as low as 1 ml to 5 ml (i.e. an equivalent of 1 mg of tablet composition per 0.01 ml to 0.005 ml water). In this instance, the resultant mixture may be described as a dispersion but also as a soft mass. For such a soft mass, this may not pass through the above-mentioned sieve screen (given the low volume of water with which it is mixed) but may nevertheless be suitable for administration, i.e. that soft mass may suitably be administered by spoon. Equally, the composition may be administered by mixing with another suitable medium or vehicle for administration (as described above), which may be a dispersion, soft mass (if e.g. the composition is only mixed with a relatively small volume of water) or another mixture (e.g. the composition with a semi-solid).

In contrast, the formulation disclosed in the Examples in WO 2008/068231 does not have comparable dispersibility/disintegrating properties. In particular, such prior formulations may not have comparable dispersibility properties, and more particularly they (e.g. a 100 mg composition in 50 mL of water, or equivalent compositions by weight to volume) may disperse in a time of greater than 90 seconds, e.g. greater than 120 seconds (and may disperse in greater than 180 seconds, e.g. around 240 seconds or even longer). Such relatively long dispersion times may be disadvantageous and may not be desirable.

By "evenly dispersed", we mean that the composition (e.g. tablet composition) rapidly disintegrates in water into physically smaller particles that are spread out (or dispersed) throughout the water. This results in any equal portion of the water containing approximately equal amounts of composition (e.g. tablet composition) particles (by weight), by which we mean within a deviation of ±25%, preferably ±15%, and especially ±10% (or less e.g. within ±5%). Hence, if a 100 mg tablet composition is dispersed in 50 ml of water, then each portion of 25 ml of water (when divided) should contain about 50 mg of tablet composition weight, but with a possible deviation of ±25% (i.e. ±12.5 mg), preferably, ±15% (i.e. ± 7.5 mg) and especially ±10% (i.e. ± 5 mg) - most preferably the deviation will be ±5% (i.e. ±2.5 mg). Hence, the tablet compositon is physically uniform or homogenous throughout the water medium in which it is placed (after the necessary time for dispersion; see above). It will be understood that the larger the volume of water per mg of tablet composition, the less deviation there may be in terms of dispersion.

Where it is indicated that the water-dispersible composition (e.g. tablet composition) may pass through a 710 µm sieve, this is in order that the dispersible composition (e.g. tablet) meets certain quality thresholds/requirements, for example those in the current (or future) editions of the British Pharmacopoeia and European Pharmacopoeia. Hence why the dispersion quality (passing through the sieve) is important, as well as the dispersion time (most preferably dispersion within 30 seconds). Although these properties are important for an actual dispersion in aqueous media, it will be understood that a dispersion (e.g. in water) need not be prepared, but the dispersible tablet may be administered in alternative ways. For example, the dispersible tablet may be mixed with certain foods (as such or by forming a soft mass by mixing the tablet composition with a small quanity/volume of water as described above). This is elaborated upon below.

The dispersible composition (e.g. tablet) of the invention will now be described in more details. Clearly, it has intrinsic properties that allow for the dispersibility (or disintegration) properties.

Hence in an aspect of the invention there is provided a dispersible composition (e.g. tablet) comprising bedaquiline fumarate as the active ingredient and wherein the tablet comprises an intra-granular and extra-granular layer in which the intra-granular layer comprises a non-soluble excipient/diluent and is charaterised in that the intra-granular layer is absent a soluble excipient/diluent that is starch (and in the most preferred embodiment the intra-granular layer is absent any soluble excipient/diluent). For example the intra-granular layer may comprise mannitol as an excipient/diluent (which is classed as a soluble excipient/diluent) but may not contain starch. In the preferred embodiment, the intra-granular layer is absent mannitol and starch (and also absent any other soluble excipient/diluent).

In an alternative aspect of the invention, the intra-granular layer comprises a non-soluble excipient/diluent that is microcrystalline cellulose, but such layer in not necessarily absent a soluble excipient/diluent.

In an embodiment, when a soluble excipient/diluent is employed, then it is preferably not starch. This is because starch may swell in water (e.g. by about 5-10%) at 37°C (it may become soluble in hot water at temperatures above the gelatinization temperature.

It is preferred that the intra-granular layer comprises a non-soluble excipient/diluent that is microcrystalline cellulose and that layer is also absent any soluble excipient/diluent.

When microcrystalline cellulose is referred to, it is intended to include silicified microcrystalline cellulose. This non-soluble excipient/diluent in the intra-granular portion of the tablet of the invention is key to its intrinsic dispersibility/disintegrating properties.

By "absent", in the context of soluble excipient/diluent, we mean that the composition (e.g. tablet composition) contains an insignificant amount of such ingredient (e.g. in this case soluble excipient/diluent), by which we mean less than 5 % by weight based on the total weight of the composition, more preferably, less than 2.5% by weight, e.g. less than 1%. Most preferably, this means that the ingredient is completely absent, i.e. that there is 0% or near 0% of that ingredient (by weight) - that is a negligible amount of it.

Often, dispersible tablets are manufactured with soluble excipients/diluents, for example sugar-based excipients such as xylitol, fructose, lactose, and the like. However, in this case it was found that soluble excipients were disadvantageous to the dispersibility/disintegrating properties (as indicated in a reference example hereinafter), for example, due to the fact that they may take up water and form a saturated layer preventing further diffusion of solute from the saturated stagnant layer (as per Noyes Whitney's diffusion layer theory) - this phenomenon may be the cause of the adverse impact on the desired dispersion time. It may also be that the soluble excipients are more prone to absorbing environmental moisture. This may also occur if soluble excipients are used in combination with with partly soluble, or insoluble, excipient. In any event, it was clear that the use of a non-soluble excipient, in particular microcryalline cellulose, especially within the intra-granular part of the tablet formulation was key in obtaining a dispersible tablet with the desired properties.

Hence, in an aspect of the invention there is provided a dispersible composition (e.g. tablet composition) comprising (e.g. consisting of) by weight based on the total weight of the composition:
5 to 50% (e.g. 10 to 30%) of active ingredient
35% to 90% (e.g. 50 to 70%) of a non-soluble excipient/diluent
2% to 10% (e.g. 4 to 8%) of a disintegrant
0.1 to 5% (e.g. 1.5 to 3.5%) of a glidant
0.01 to 5% (e.g. 0.1 to 1%) of a wetting agent or surfactant
0 to 10% (e.g. 2 to 5%) of a binder or polymer
0 to 5% (e.g. 1 to 3%) of a lubricant
solvent (qs) e.g. water
which may also be referred to as a composition of the invention.

In the case of the above composition, where the non-soluble excipient is microcrystalline cellulose (such as silicified microcrystalline cellulose), then the quantity by weight may be between 20% to 90%, with the remaining amounts of the composition as defined herein.

The compositions of the invention mentioned herein may be charactersied in that they are absent a soluble excipient/diluent.

For instance, most preferably the composition (e.g. tablet composition) consists of by weight, based on the total weight of the composition:
24.18% (or about 25%) of active ingredient
62.12% (or about 60%) non-soluble excipient/diluent (e.g. microcrystalline cellulose, such as silicified microcrystalline cellulose)
6% (or about 6%) disintegrant (e.g. crospovidone, such as Polyplasdone XL)
2.5 % (or about 2.5%) glidant (e.g. colloidal silicon dioxide, Aerosil 200)
0.2% (or about 0.2%) wetting agent or surfactant (e.g. polysorbate 20, i.e. Tween 20)
3% (or about 3%) binder or polymer (e.g. hypromellose 5 cps, i.e. Methocel E 5 LV)
2% (or about 2%) lubricant (e.g. sodium stearyl fumarate (Pruv))
Solvent (qs), e.g. water - if necessary (i.e. only the amount needed, if any)

In another aspect, there is provided a composition (e.g. tablet composition) wherein the different parts of the composition, specifically the intra-granular and extra-granular fraction and binder portion, comprise (e.g. consist of) the following ingredients by weight based on the total weight of the composition:
Intra-granular fraction
   5 to 50% (e.g. 10 to 30%) of active ingredient
   10 to 50% (e.g. 20 to 40%) of non-soluble excipient/diluent (e.g. microcrystalline cellulose, such as silicified microcrystalline cellulose)
   1 to 5% (e.g. 2 to 4%) disintegrant (e.g. crospovidone, such as Polyplasdone XL)
   0.1 to 5% (e.g. 0.5 to 4%, such as 1 to 3%) glidant (e.g. colloidal silicon dioxide, Aerosil 200)
Binder
   1 to 10% (e.g. 2 to 5%) 3% binder or polymer (e.g. hypromellose 5 cps, i.e. Methocel E 5 LV)
   0.01 to 5% (e.g. 0.1 to 1%) wetting agent or surfactant (e.g. polysorbate 20, i.e. Tween 20)
   Solvent (qs), e.g. water - if necessary (i.e. only the amount needed, if any)
Extra-granular fraction
   10 to 50% (e.g. 20 to 40%) of excipient/diluent (preferably a non-soluble excipient/diluent e.g. microcrystalline cellulose, such as silicified microcrystalline cellulose)
   1 to 5% (e.g. 2 to 4%) disintegrant (e.g. crospovidone, such as Polyplasdone XL)
   0 to 3% (e.g. 0.1 to 1%) glidant (e.g. colloidal silicon dioxide, Aerosil 200)
   0 to 5% (e.g. 1 to 3%) lubricant (e.g. sodium stearyl fumarate (Pruv))

The compositions (e.g. tablet compositions) of the invention mentioned herein may be charactersied in that specifically the intra-granular layer is absent any soluble excipient/diluent. Hence, the extra-granular fraction need not be absent any soluble excipient/diluent, although, preferably, it is the case that the extra-granular fraction is also absent any soluble excipient/diluent.

For instance, most preferably the composition consists of the following compositions of intra-granular fraction, binder and extra-granular fraction, by weight based on the total weight of the composition:
Intra-granular fraction
   24.18% (or about 25%) of active ingredient
   29.82% (or about 30%) of non-soluble excipient/diluent (e.g. microcrystalline cellulose, such as silicified microcrystalline cellulose)
   3% (or about 3%) disintegrant (e.g. crospovidone, such as Polyplasdone XL)
   2 % (or about 2%) glidant (e.g. colloidal silicon dioxide, Aerosil 200)
Binder
   3% (or about 3%) binder or polymer (e.g. hypromellose 5 cps, i.e. Methocel E 5 LV)
   0.2% (or about 0.2%) wetting agent or surfactant (e.g. polysorbate 20, i.e. Tween 20)
   Solvent (qs), e.g. water - if necessary (i.e. only the amount needed, if any)
Extra-granular fraction
   32.3% (or about 30%) of excipient/diluent (preferably a non-soluble excipient/diluent e.g. microcrystalline cellulose, such as silicified microcrystalline cellulose)
   3% (or about 3%) disintegrant (e.g. crospovidone, such as Polyplasdone XL)
   0.5 % (or about 0.5%) glidant (e.g. colloidal silicon dioxide, Aerosil 200)
   2% (or about 2%) lubricant (e.g. sodium stearyl fumarate (Pruv))

The intra-granular fraction (or portion) may comprise up to 75% by weight of the total weight of the composition (e.g. tablet), and preferably comprises between 40 and 70% (e.g. between 50 and 65%) by weight of the composition (or tablet). Most preferably, the intra-granular fraction (or portion) of the composition (e.g. tablet composition) comprises about 60% of the total weight of the composition. The binder fraction (or portion, or element) may comprise up to 20% by weight of the total weight of the composition (e.g. tablet composition), for example from 0.5 to 10% by weight and preferably between 1 and 8% by weight (e.g. about 3% by weight). The extra-granular layer may comprise up to 60% by weight of the total weight of the composition (e.g. tablet), and preferably comprises between 20 and 50% (e.g. between 30 and 45%) by weight of the composition (or tablet). Most preferably, the extra-granular fraction (or portion) of the composition (e.g. tablet composition) comprises about 37.5% of the total weight of the composition.

In the context of the compositions (e.g.tablet compositions) described herein, the aspects of the composition may be described as comprising an intra-granular and extra-granular fraction and a binder portion (or fraction). Such fractions or portions of the composition are ultimately intermingled with each other. However, it will be appreciated (for example with reference to the process for preparing such compositions) that the distinction of these fractions (or portions) results in distinct properties for the resultant compositions.

The compositions of the invention described herein may be a mixture of or blend of the intra-granular and extra-granular fractions (or portions) and binder portion and may also, after being subjected to a suitable compression technique, take on a (unit) dosage form such as a tablet.

In aspects of the invention described herein, particularly the dispersible compositions described above, the total tablet weight may be about 100 mg (and hence, the active ingredient present may be between 5 to 50 mg, such as between about 10 mg and 30 mg, e.g. about 20 mg). In this manner a pediatric (or geriatric) formulation may be provided in which there is about 20 mg of active ingredient. In other aspects, particularly those described below, the total tablet weight may be higher (but may still deliver the same quantity of active ingredient), for instance, a dispersible formulation of 200 mg may be provided to also deliver about 20 mg of active ingredient, for instance in the aspects and percentages that may be described below.

In another aspect of the invention, there may also be provided dispersible compositions with the following features, which applies in particular to compositions of the invention where the total tablet weight is relatively high (for instance greater than 100 mg, e.g. a total tablet weight of 200 mg):
- there is provided a dispersible composition (e.g. tablet composition) comprising (e.g. consisting of) by weight based on the total weight of the composition: 5 to 50% (e.g. 5 to 20% or 10 to 15%) of active ingredient 35% to 90% (e.g. 60 to 80% or 70 to 75%) of a non-soluble excipient/diluent 2% to 10% (e.g. 4 to 8%) of a disintegrant
   0.1 to 5% (e.g. 1.5 to 3.5%) of a glidant
   0.01 to 5% (e.g. 0.1 to 1%) of a wetting agent or surfactant
   0 to 10% (e.g. 2 to 5%) of a binder or polymer
   0 to 5% (e.g. 1 to 3%) of a lubricant
   solvent (qs) e.g. water
- there is provided a dispersible composition (e.g. tablet composition) comprising (e.g. consisting of) by weight based on the total weight of the composition:
   12.09% (or about 12%) of active ingredient
   73.71% (or about 70%) non-soluble excipient/diluent (e.g. microcrystalline cellulose, such as silicified microcrystalline cellulose)
   6% (or about 6%) disintegrant (e.g. crospovidone, such as Polyplasdone XL)
   2.5 % (or about 2.5%) glidant (e.g. colloidal silicon dioxide, Aerosil 200)
   0.2% (or about 0.2%) wetting agent or surfactant (e.g. polysorbate 20, i.e. Tween 20)
   3% (or about 3%) binder or polymer (e.g. hypromellose 5 cps, i.e. Methocel E 5 LV)
   2% (or about 2%) lubricant (e.g. sodium stearyl fumarate (Pruv))
   Solvent (qs), e.g. water - if necessary (i.e. only the amount needed, if any)

In another aspect, in particular to those compositions of the invention where the total tablet weight is relatively high (e.g. 200 mg):
- there is provided a composition (e.g. tablet composition) wherein the different parts of the composition, specifically the intra-granular and extra-granular fraction and binder portion, comprise (e.g. consist of) the following ingredients by weight based on the total weight of the composition:
   Intra-granular fraction
      5 to 50% (e.g. 5 to 20% or 10 to 15%) of active ingredient
      10 to 50% (e.g. 30 to 50% or 35 to 45%) of non-soluble excipient/diluent (e.g. microcrystalline cellulose, such as silicified microcrystalline cellulose)
      1 to 5% (e.g. 2 to 4%) disintegrant (e.g. crospovidone, such as Polyplasdone XL)
      0.1 to 5% (e.g. 0.5 to 4%, such as 1 to 3%) glidant (e.g. colloidal silicon dioxide, Aerosil 200)
   Binder
      1 to 10% (e.g. 2 to 5%) 3% binder or polymer (e.g. hypromellose 5 cps, i.e. Methocel E 5 LV)
      0.01 to 5% (e.g. 0.1 to 1%) wetting agent or surfactant (e.g. polysorbate 20, i.e. Tween 20)
      Solvent (qs), e.g. water - if necessary (i.e. only the amount needed, if any)
   Extra-granular fraction
      10 to 50% (e.g. 20 to 40%) of excipient/diluent (preferably a non-soluble excipient/diluent e.g. microcrystalline cellulose, such as silicified microcrystalline cellulose)
      1 to 5% (e.g. 2 to 4%) disintegrant (e.g. crospovidone, such as Polyplasdone XL)
      0 to 3% (e.g. 0.1 to 1%) glidant (e.g. colloidal silicon dioxide, Aerosil 200)
      0 to 5% (e.g. 1 to 3%) lubricant (e.g. sodium stearyl fumarate (Pruv))
- there is provided provided a composition (e.g. tablet composition) wherein the different parts of the composition, specifically the intra-granular and extra-granular fraction and binder portion, comprise (e.g. consist of) the following ingredients by weight based on the total weight of the composition:
   Intra-granular fraction
      12.09% (or about 12%) of active ingredient
      41.41% (or about 40%) of non-soluble excipient/diluent (e.g. microcrystalline cellulose, such as silicified microcrystalline cellulose)
      3% (or about 3%) disintegrant (e.g. crospovidone, such as Polyplasdone XL)
      2 % (or about 2%) glidant (e.g. colloidal silicon dioxide, Aerosil 200)
   Binder
      3% (or about 3%) binder or polymer (e.g. hypromellose 5 cps, i.e. Methocel E 5 LV)
      0.2% (or about 0.2%) wetting agent or surfactant (e.g. polysorbate 20, i.e. Tween 20)
      Solvent (qs), e.g. water - if necessary (i.e. only the amount needed, if any)
   Extra-granular fraction
      32.3% (or about 30%) of excipient/diluent (preferably a non-soluble excipient/diluent e.g. microcrystalline cellulose, such as silicified microcrystalline cellulose)
      3% (or about 3%) disintegrant (e.g. crospovidone, such as Polyplasdone XL)
      0.5 % (or about 0.5%) glidant (e.g. colloidal silicon dioxide, Aerosil 200)
      2% (or about 2%) lubricant (e.g. sodium stearyl fumarate (Pruv))

The compositions of the invention are described as having certain components or ingredients, which is elaborated below.

By "active ingredient" we mean bedaquiline fumarate, i.e. the fumarate salt form of bedaquiline. This is the form that is a part of the adult composition that has been received regulatory approval in some territories.

It is indicated herein that the compositions (e.g. tablet compositions) of the invention contain a non-soluble excipient/diluent. Unless that excipient is already specified, such excipient/diluent may be starch, powdered cellulose, microcrystalline cellulose (such as silicified microcrystalline cellulose), calcium phosphates (e.g. dibasic calcium phosphate, dibasic calcium phosphate dihydrate, tribasic calcium phosphate), calcium carbonate, calcium sulfate or the like (or combinations thereof, i.e. co-processed non-soluble excipients; others that may be considered include wax-like hydrogenated oils and the like). It is understood that the most preferred non-soluble excipient/diluent is microcrystalline cellulose (e.g. silicified microcrystalline cellulose) because this results in compositions with intrinsic properties that are advantageous. Where it is mentioned that the excipient/diluent need not be non-soluble, then sugars and polyols may also be considered, for instance the following excipients/diluents may also be considered: dextrates, dextrin, dextrose excipient, fructose, kaolin, lactitol, lactose anhydrous, lactose monohydrate, mannitol, sorbitol, sodium chloride, sucrose, compressible sugar, confectioner's sugar, a spray-dried mixture of lactose monohydrate and microcrystalline cellulose (75:25), commercially available as Microcelac^{®}, a co-processed spray-dried mixture of microcrystalline cellulose and colloidal silicon dioxide (98:2), commercially available as Prosolv^{®} (which possibilities include sugars and other soluble excipients/diluents).

It is indicated herein that the compositions (e.g. tablet compositions) of the invention contain a disintegrant. Possible disintegrants include pharmaceutically acceptable disintegrants comprising starch, ion exchange resins, e.g. Amberlite, cross-linked polyvinylpyrrolidone, modified cellulose gum, e.g. croscarmellose sodium (e.g. Ac-di-Sol^{®}), sodium starch glycollate, sodium carboxymethylcellulose, sodium dodecyl sulphate, modified corn starch, microcrystalline cellulose, magnesium aluminium silicate, alginic acid, alginate, powdered cellulose, crospovidone (such as Polyplasdone XL). Other disintegrants that may be considered include L-HPC, Xanthan gum, Gellan gum, soy polysaccharides, and the like. The most preferred disintegrant is crospovidone, preferably a coarse grade crospovidone (such as Polyplasdone XL).

It is indicated herein that the compositions (e.g. tablet compositions) of the invention contain a glidant. Possible glidants include pharmaceutically acceptable glidants comprising talc, colloidal silicon dioxide, starch, magnesium stearate. Preferred is colloidal silicon dioxide (Aerosil 200)

It is indicated herein that the compositions (e.g. tablet compositions) of the invention contain a wetting agent or surfactant. Such wetting agent (or surfactant) may be any of the physiologically tolerable wetting agents suitable for use in a pharmaceutical composition.

It is well-known in the art that a wetting agent is an amphiphilic compound; it contains polar, hydrophilic moieties as well as non-polar, hydrophobic moieties.

The terms "hydrophilic" or "hydrophobic" are relative terms.

The relative hydrophilicity or hydrophobicity of a wetting agent may be expressed by its hydrophilic-lipophilic balance value ("HLB value). Wetting agents with a lower HLB value are catagorized as being "hydrophobic" wetting agents whereas wetting agents with a higher HLB value are catagorized as being "hydrophilic" wetting agents. As a rule of thumb, wetting agents having a HLB value greater than about 10 are generally considered as being hydrophilic wetting agents; wetting agents having a HLB value lower than about 10 are generally considered as being hydrophobic wetting agents.

The present compositions preferably comprise a hydrophilic wetting agent.

It should be appreciated that the HLB value of a wetting agent is only a rough guide to indicate the hydrophilicity/hydrophobicity of a wetting agent. The HLB value of a particular wetting agent may vary depending upon the method used to determine the HLB value; may vary depending on its commercial source; is subject to batch to batch variability. A person skilled in the art can readily identify hydrophilic wetting agents suitable for use in the pharmaceutical compositions of the present invention.

The wetting agent of the present invention can be an anionic, a cationic, a zwitterionic or a non-ionic wetting agent, the latter being preferred. The wetting agent of the present invention can also be a mixture of two or more wetting agents.

Suitable wetting agents for use in the compositions of the present invention are listed below. It should be emphasized that said list of wetting agents is only illustrative, representative and not exhaustive. Thus the invention is not limited to the wetting agents listed below. In the present compositions, also mixtures of wetting agents may be used.

Suitable wetting agents which may be used in the present invention comprise :
a) Polyethylene glycol fatty acid monoesters comprising esters of lauric acid, oleic acid, stearic acid, ricinoic acid and the like with PEG 6, 7, 8, 9, 10, 12, 15, 20, 25, 30, 32, 40, 45, 50, 55, 100, 200, 300, 400, 600 and the like, for instance PEG-6 laurate or stearate, PEG-7 oleate or laurate, PEG-8 laurate or oleate or stearate, PEG-9 oleate or stearate, PEG-10 laurate or oleate or stearate, PEG-12 laurate or oleate or stearate or ricinoleate, PEG-15 stearate or oleate, PEG-20 laurate or oleate or stearate, PEG-25 stearate, PEG-32 laurate or oleate or stearate, PEG-30 stearate, PEG-40 laurate or oleate or stearate, PEG-45 stearate, PEG-50 stearate, PEG-55 stearate, PEG-100 oleate or stearate, PEG-200 oleate, PEG-400 oleate, PEG-600 oleate; (the wetting agents belonging to this group are for instance known as Cithrol, Algon, Kessco, Lauridac, Mapeg, Cremophor, Emulgante, Nikkol, Myrj, Crodet, Albunol, Lactomul)
b) Polyethylene glycol fatty acid diesters comprising diesters of lauric acid, stearic acid, palmic acid, oleic acid and the like with PEG-8, 10, 12, 20, 32, 400 and the like, for instance PEG-8 dilaurate or distearate, PEG-10 dipalmitate, PEG-12 dilaurate or distearate or dioleate, PEG-20 dilaurate or distearate or dioleatePEG-32 dilaurate or distearate or dioleate, PEG-400 dioleate or distearate; (the wetting agents belonging to this group are for instance known as Mapeg, Polyalso, Kessco, Cithrol)
c) Polyethylene glycol fatty acid mono-and diester mixtures such as for example PEG 4-150 mono and dilaurate, PEG 4-150 mono and dioleate, PEG 4-150 mono and distearate and the like; (the wetting agents belonging to this group are for instance known as Kessco)
d) Polyethylene glycol glycerol fatty acid esters such as for instance PEG-20 glyceryl laurate or glyceryl stearate or glyceryl oleate, PEG-30 glyceryl laurate or glyceryl oleate, PEG-15 glyceryl laurate, PEG-40 glyceryl laurate and the like; (the wetting agents belonging to this group are for instance known as Tagat, Glycerox L, Capmul) ,
e) Alcohol-oil transesterification products comprising esters of alcohols or polyalcohols such as glycerol, propylene glycol, ethylene glycol, polyethylene glycol, sorbitol, pentaerythritol and the like with natural and/or hydrogenated oils or oil-soluble vitamins such as castor oil, hydrogenated castor oil, vitamin A, vitamin D, vitamin E, vitamin K, an edible vegetable oil e.g. corn oil, olive oil, peanut oil, palm kernel oil, apricot kernel oil, almond oil and the like, such as PEG-20 castor oil or hydrogenated castor oil or corn glycerides or almond glycerides, PEG-23 castor oil, PEG-25 hydrogenated castor oil or trioleate, PEG-35 castor oil, PEG-30 castor oil or hydrogenated castor oil, PEG-38 castor oil, PEG-40 castor oil or hydrogenated castor oil or palm kernel oil, PEG-45 hydrogenated castor oil, PEG-50 castor oil or hydrogenated castor oil, PEG-56 castor oil, PEG-60 castor oil or hydrogenated castor oil or corn glycerides or almond glycerides, PEG-80 hydrogenated castor oil, PEG-100 castor oil or hydrogenated castor oil, PEG-200 castor oil, PEG-8 caprylic/capric glycerides, PEG-6 caprylic/capric glycerides, lauroyl macrogol-32 glyceride, stearoyl macrogol glyceride, tocopheryl PEG-1000 succinate (TPGS); (the wetting agents belonging to this group are for instance known as Emalex, Cremophor, Emulgante, Eumulgin, Nikkol, Thornley, Simulsol, Cerex, Crovol, Labrasol, Softigen, Gelucire, Vitamin E TPGS),
f) polyglycerized fatty acids comprising polyglycerol esters of fatty acids such as for instance polyglyceryl-10 laurate or oleate or stearate, polyglyceryl-10 mono and dioleate, polyglyceryl polyricinoleate and the like; (the wetting agents belonging to this group are for instance known as Nikkol Decaglyn, Caprol or Polymuls)
g) Sterol derivatives comprising polyethylene glycol derivatives of sterol such as PEG-24 cholesterol ether, PEG-30 cholestanol, PEG-25 phyto sterol, PEG-30 soya sterol and the like; (the wetting agents belonging to this group are for instance known as Solulan^{™} or Nikkol BPSH)
h) Polyethylene glycol sorbitan fatty acid esters such as for example PEG-10 sorbitan laurate, PEG-20 sorbitan monolaurate or sorbitan tristearate or sorbitan monooleate or sorbitan trioleate or sorbitan monoisostearate or sorbitan monopalmiate or sorbitan monostearate, PEG-4 sorbitan monolaurate, PEG-5 sorbitan monooleate, PEG-6 sorbitan monooleate or sorbitan monolaurate or sorbitan monostearate, PEG-8 sorbitan monostearate, PEG-30 sorbitan tetraoleate, PEG-40 sorbitan oleate or sorbitan tetraoleate, PEG-60 sorbitan tetrastearate, PEG-80 sorbitan monolaurate, PEG sorbitol hexaoleate (Atlas G-1086) and the like; (the wetting agents belonging to this group are for instance known as Liposorb, Tween, Dacol MSS, Nikkol, Emalex, Atlas)
i) Polyethylene glycol alkyl ethers such as for instance PEG-10 oleyl ether or cetyl ether or stearyl ether, PEG-20 oleyl ether or cetyl ether or stearyl ether, PEG-9 lauryl ether, PEG-23 lauryl ether (laureth-23), PEG-100 stearyl ether and the like; (the wetting agents belonging to this group are for instance known as Volpo, Brij)
j) Sugar esters such as for instance sucrose distearate/monostearate, sucrose monostearate or monopalmitate or monolaurate and the like; (the wetting agents belonging to this group are for instance known as Sucro ester, Crodesta, Saccharose mono laurate)
k) Polyethylene glycol alkyl phenols such as for instance PEG-10-100 nonyl phenol (Triton X series), PEG-15-100 ocyl phenol ether (Triton N series) and the like;
l) Polyoxyethylene-polyoxypropylene block copolymers (poloxamers) such as for instance poloxamer 108, poloxamer 188, poloxamer 237, poloxamer 288 and the like; (the wetting agents belonging to this group are for instance known as Synperonic PE, Pluronic, Emkalyx, Lutrol^{™}, Supronic, Monolan, Pluracare, Plurodac)
m) ionic wetting agents including cationic, anionic and zwitterionic surfactans such as the fatty acid salts e.g. sodium oleate, sodium lauryl sulfate, sodium lauryl sarcosinate, sodium dioctyl sulfosuccinate, sodium myristate, sodium palmitate, sodium state, sodium ricinoleate and the like; such as bile salts e.g. sodium cholate, sodium taurocholate, sodium glycocholate and the like; such as phospholipids e.g. egg/soy lecithin, hydroxylated lecithin, lysophosphatidylcholine, phosphatidylcholine, phosphatidyl ethanolamine, phosphatidyl glycerol, phosphatidyl serine and the like; such as phosphoric acid esters e.g. diethanolammonium polyoxyethylene-10 oleyl ether phosphate, esterification products of fatty alcohols or fatty alcohol ethoxylates with phosphoric acid or anhydride; such as carboxylates e.g. succinylated monoglycerides, sodium stearyl fumarate, stearoyl propylene glycol hydrogen succinate, mono/diacetylated tartaric acid esters of mono-and diglycerides, citric acid esters of mono-and diglycerides, glyceryl-lacto esters of fatty acids, lactylic esters of fatty acids, calcium/sodium stearoyl-2-lactylate, calcium/sodium stearoyl lactylate, alginate salts, propylene glycol alginate, ether carboxylates and the like; such as sulfates and sulfonates e.g. ethoxylated alkyl sulfates, alkyl benzene sulfates, alpha-olefin sulfonates, acyl isethionates, acyl taurates, alkyl glyceryl ether sulfonates, octyl sulfosuccinate disodium, disodium undecyleneamido-MEA-sulfosuccinate and the like; such as cationic wetting agents e.g. hexadecyl triammonium bromide, decyl trimethyl ammonium bromide, cetyl trimethyl ammonium bromide, dodecyl ammonium chloride, alkyl benzyldimethylammonium salts, diisobutyl phenoxyethoxydimethyl benzylammonium salts, alkylpyridinium salts, betaines (lauryl betaine), ethoxylated amines (polyoxyethylene-15 coconut amine) and the like.

When in the above list of suitable wetting agents, different possibilities are listed such as for example PEG-20 oleyl ether or cetyl ether or stearyl ether, this means that PEG-20 oleyl ether and PEG-20 cetyl ether and PEG-20 stearyl ether are intended. Thus for instance PEG-20 castor oil or hydrogenated castor oil or corn glycerides or almond glycerides has to be read as PEG-20 castor oil and PEG-20 hydrogenated castor oil and PEG-20 corn glycerides and PEG-20 almond glycerides.

Preferred wetting agents in the present compositions are those agents belonging to the group of the polyethylene glycol sorbitan fatty acid esters, such as wetting agents known as Tween, e.g. Tween 20, 60, 80. Most preferably, the wetting agent is Tween 20 (polysorbate 20).

The preferred quantity of wetting agent (or surfactant) is described herein, but it is appreciated however that when used in the present compositions, it may depend on the amount of active ingredient present in the composition or on the particle size of the active ingredient. A higher amount or a smaller particle size may require more wetting agent.

It is indicated herein that the compositions (e.g. tablet compositions) of the invention contain a binder or polymer (for instance for the binder fraction of the compositions of the invention). Such a binder or polymer may be an organic polymer.

The organic polymer used in the compositions (e.g. tablets) of the invention may be any of the physiologically tolerable water soluble synthetic, semi-synthetic or non-synthetic organic polymers.

Thus for example the polymer may be a natural polymer such as a polysaccharide or polypeptide or a derivative thereof, or a synthetic polymer such as a polyalkylene oxide (e.g. PEG), polyacrylate, polyvinylpyrrolidone, etc. Mixed polymers, e.g. block copolymers and glycopeptides may of course also be used.

The polymer conveniently has a molecular weight in the range 500D to 2 MD, and conveniently has an apparent viscosity of 1 to 15,000 mPa.s when in a 2% aqueous solution at 20°C. For example, the water-soluble polymer can be selected from the group comprising
- alkylcelluloses such as methylcellulose,
- hydroxyakylcelluloses such as hydroxymethylcellulose, hydroxyethylcellulose, hydroxypropylcellulose and hydroxybutylcellulose,
- hydroxyalkyl alkylcelluloses such as hydroxyethyl methylcellulose and hydroxypropyl methylcellulose,
- carboxyalkylcelluloses such as carboxymethylcellulose,
- alkali metal salts of carboxyalkylcelluloses such as sodium carboxymethylcellulose,
- carboxyalkylalkylcelluloses such as carboxymethylethylcellulose,
- carboxyalkylcellulose esters,
- starches,
- pectins such as sodium carboxymethylamylopectin,
- chitin derivates such as chitosan,
- heparin and heparinoids,
- polysaccharides such as alginic acid, alkali metal and ammonium salts thereof, carrageenans, galactomannans, tragacanth, agar-agar, gum arabic, guargum and xanthan gum,
- polyacrylic acids and the salts thereof,
- polymethacrylic acids and the salts thereof, methacrylate copolymers,
- polyvinylalcohol,
- polyvinylpyrrolidone, copolymers of polyvinylpyrrolidone with vinyl acetate,
- polyalkylene oxides such as polyethylene oxide and polypropylene oxide and copolymers of ethylene oxide and propylene oxide, e.g. poloxamers and poloxamines.

Non-enumerated polymers which are pharmaceutically acceptable and have appropriate physico-chemical properties as defined hereinbefore are equally suited for preparing compositions according to the present invention.

Preferably the organic polymer is starch, polyvinylpyrrolidone or a cellulose ether, e.g. PVP K29-32, PVP K90, methyl cellulose, hydroxypropylcellulose, hydroxyethyl methylcellulose, or hydroxypropyl methylcellulose (HPMC).

Said HPMC contains sufficient hydroxypropyl and methoxy groups to render it water-soluble. HPMC having a methoxy degree of substitution from about 0.8 to about 2.5 and a hydroxypropyl molar substitution from about 0.05 to about 3.0 are generally water-soluble. Methoxy degree of substitution refers to the average number of methyl ether groups present per anhydroglucose unit of the cellulose molecule. Hydroxypropyl molar substitution refers to the average number of moles of propylene oxide which have reacted with each anhydroglucose unit of the cellulose molecule. A preferred HPMC is hypromellose 2910 15 mPa.s or hypromellose 2910 5mPa.s, especially hypromellose 2910 15 mPa.s. Hydroxypropyl methylcellulose is the United States Adopted Name for hypromellose (see Martindale, The Extra Pharmacopoeia, 29th edition, page 1435). In the four digit number "2910", the first two digits represent the approximate percentage of methoxyl groups and the third and fourth digits the approximate percentage composition of hydroxypropoxyl groups; 15 mPa.s or 5 mPa.s is a value indicative of the apparent viscosity of a 2 % aqueous solution at 20°C.

It is most preferred that the binder or polymer of the compositions of the invention is hypromellose 5 cps (i.e. Methocel E 5 LV).

It is indicated herein that the compositions (e.g. tablet compositions) of the invention contain a lubricant. Such a lubricant may be pharmaceutically acceptable lubricants such as magnesium stearate, calcium stearate, stearic acid, talc, polyethylene glycol, sodium lauryl sulfate, magnesium lauryl sulphate. It is most preferred that the lubricant is sodium stearyl fumarate (Pruv).

The compositions of the invention may make use of active ingredient having a particle size of:
- d¹⁰ less than 50µm (preferably less than 25µm, for instance less than 15µm, e.g. around 9µm (or even less)
- d⁵⁰ less than 100µm (preferably less than 50µm, for instance less than 25µm, e.g. around 22µm (or even less)
- d⁹⁰ less than 200µm (preferably less than 100µm, for instance less than 50µm, e.g. around 48µm (or even less)

As used herein, the term d⁵⁰ has its conventional meaning as known to the person skilled in the art and can be measured by art-known particle size measuring techniques such as, for example, sedimentation field flow fractionation, photon correlation spectroscopy, laser diffraction or disk centrifugation. The d⁵⁰ mentioned herein may be related to volume distributions of the particles. In that instance, by "a d⁵⁰ of 22 µm" it is meant that at least 50% of the volume of the particles has a particle size of less than 22 µm. The same applies to the other particle sizes mentioned and d¹⁰ and d⁹⁰ have analogous meanings. Usually volume and weight distribution result in the same or about the same value for the average particle size.

The particle size can be an important factor determining the tabletting speed, in particular the flowability and therefore the manufacturability on a large scale of a particular dosage form or formulation, and the quality of the final product. For instance, for capsules, the particle size may range preferably from about 5 to about 300 µm (d⁵⁰); for tablets the particle size is preferably less than 250 µm, more preferably less than 100 µm (e.g. less than 50 µm) (d⁵⁰). Too small particles can cause sticking on the tablet punches and manufacturability issues. The particle size has an effect on the intrinsic properties of the compositions of the invention.

The compositions as described herein may further comprise one or more pharmaceutically acceptable excipients such as, for example, plasticizers, flavours, sweeteners, coluorants, preservatives and the like (provided that such additional excipients do not comprise soluble excipients/diluents, where it is already specified that the composition or fraction of that composition, as appropriate, does not comprise such soluble components). In an aspect of the invention, the compositions of the invention do not contain a plasticizer or another such optional excipient mentioned here. Especially in case of preparation by hot melt extrusion, said excipients should not be heat-sensitive, in other words, they should not show any appreciable degradation or decomposition at the working temperature of the melt-extruder.

The advantage of the present combinations of the invention, and specifically the dispersibility/disintegrating properties, may stem from the presence of the non-soluble excipient (e.g. microcrystalline cellulose, such as silicified microcrystalline cellulose) which may have the ability to rapidly intake water in the dispersion medium, i.e. it may have a wicking action that advantageously results in the improved dispersibility/disintegrating properties. In view of this, advantageously, the compositions of the invention achieve a favourable dispersibiltiy or disintegrating action, for example as compared to compositions previously known or as compared to other compositions as may be described herein. As indicated, wicking action may lead to faster dispersion and may by-pass the solubilization process (for instance, liquid may be drawn up or "wicked" into these pathways through capilliary action and rupture the interparticulate bonds, causing the tablet/composition to break apart), which could be advantageous. Further, the non-soluble excipients/diluents may easily be re-suspended even after a long period of time (e.g. 6 hours) - this may have the advantage that the compositions of the invention do not require a suspending agent to re-disperse the granules/particles.

The invention also relates to process for preparing the compositions of the invention (e.g. the tablet compositions) and there is therefore provided:
- A process (e.g. as described hereinbelow) for the preparation of composition (e.g. tablet) of the invention
- A product (e.g. composition to the invention, e.g. a dispersible tablet as described herein) obtainable by a process of the invention (e.g. as described hereinbelow)

As indicated above, the compositions of the invention preferably comprise different fractions/portions, an intra-granular fraction, a binder portion and an extra-granular fraction.

Hence, there is provided a process for the preparation of a process of the invention, which comprises:
(a) obtaining an intra-granular fraction using the intra-granular fraction components mentioned herein;
(b) preparing a binder fraction, using the binder fraction components mentioned herein;
(c) obtaining an extra-granular fraction using the extra-granular fraction components mentioned herein,
and using those fractions to prepare a composition of the invention.

More specifically, the intra-granular fraction (as defined herein) may be prepared by mixing or blending the relevant components.

Direct compression may be employed but this may have the disadvantage that the blend has poor flow properties and/or there may be sticking on the surface of punches. Hence, direct compression was followed by granulation. Dry granulation may have disadvantages in relation to flow (or compression) properties and the aforementioned sticking/picking phenomenon may also remain. Hence, for compositions of the invention, it is preferred that a wet granulation process is employed.

More specifically therefore, the binder fraction/portion, may be prepared by contacting or mixing the relevant ingredients (i.e. the binder or polymer and wetting agent and, if necessary, a vehicle which may be aqueous or non-aqueous, or a combination; the vehicle is preferably water (qs), preferably purified water (qs)), and that binder fraction/portion may undergo a wet-granulation with the intra-granular fraction. Such wet granulation process is preferably a low shear granulation process (or top spray fluid bed granulation) and, in the binder fraction, a low viscosity soluble polymer (preferably viscosity 5 cps or lower) is employed. The obtained granulate may then be dried and sized (or sieved) after which it is mixed or blended with the components of the extra-granular fraction (as defined herein). Such blending also inherently involves lubrication, if the extra-granular layer also includes a lubricant.

Once a composition of the invention has been prepared, for example as set out above, including the mixing of the intra-granular, extra-granular and binder fractions, such composition may optionally, and preferably, be converted into tablet forms. In a preferred aspect of the process of the invention, the compositions so prepared are preferably compressed into tablet form, thereby allowing for the preparation of a dispersible tablet of the invention. Such a tablet may be of any suitable dose, but each unit may contain between 5 and 200 mg of active ingredient (in this instance, meaning the active substatnce bedaquiline not considering the fumarate salt component). The unit may contain 100 mg of bedaquiline (plus the corresponding weight of the fumarate salt portion) or, if the unit form is for the pediatric population, then it is preferably 20 mg of bedaquiline (corresponding to 24.18 mg of bedaquiline fumarate).

The tabletting process itself is otherwise standard and readily practised by forming a tablet from desired blend or mixture of ingredients into the appropriate shape using a conventional tablet press.

Tablets of the present invention may further be film-coated to improve taste, to provide ease of swallowing and an elegant appearance. Many suitable polymeric film-coating materials are known in the art. A preferred film-coating material is hydroxypropyl methylcellulose HPMC, especially HPMC 2910 5 mPa.s. Other suitable film-forming polymers also may be used herein, including, hydroxypropylcellulose, and acrylate-methacrylate copolymers. Besides a film-forming polymer, the film coat may further comprise a plasticizer (e.g. propylene glycol) and optionally a pigment (e.g. titanium dioxide). The film-coating suspension also may contain talc as an anti-adhesive. In immediate release tablets according to the invention, the film coat is small and in terms of weight accounts for less than about 3 % (w/w) of the total tablet weight. In an embodiment of the invention (e.g. in a preferred embodiment), the tablets of the invention are not film-coated.

As indicated above, the utility of the invention arises from the active ingredient, and salt thereof, being known to show activity against *Mycobacteria* including drug resistant strains, in particular *Mycobacterium tuberculosis, M. bovis, M. avium, M. leprae and M. marinum,* especially against *Mycobacterium tuberculosis,* including drug-resistant *M. tuberculosis* strains. The active ingredient, including salt thereof, shows activity against active, sensitive, susceptible *Mycobacteria* strains and latent, dormant, persistent *Mycobacteria* strains.

Hence, in an aspect of the invention, there is provided compositions (e.g. tablets) according to the invention, which are suitable for the treatment of a bacterial infection including a mycobacterial infection, particularly those diseases caused by pathogenic mycobacteria such as *Mycobacterium tuberculosis* (including the latent and drug resistant form thereof), *M. bovis, M. leprae, M. avium, M. leprae* and *M. marinum.*

Further, the present invention also relates to the use of a composition (e.g. tablet) of the invention, the pharmaceutically acceptable salts thereof, the solvates thereof or the *N-*oxide forms thereof, as well as any of the pharmaceutical compositions thereof as described hereinafter for the manufacture of a medicament for the treatment of a bacterial infection including a mycobacterial infection.

Accordingly, in another aspect, the invention provides a method of treating a patient suffering from, or at risk of, a bacterial infection, including a mycobacterial infection, which comprises administering to the patient a therapeutically effective amount of a pharmaceutical composition (e.g. tablet) according to the invention.

The compositions of the invention may be combined with other therapeutic agents that are known to be useful in the treatment of a bacterial infection as defined herein (and particularly for the treatment of a mycobacterial infection, tuberculosis as defined herein). Such other antibacterial agents comprise antibiotics of the β-lactam group such as natural penicillins, semisynthetic penicillins, natural cephalosporins, semisynthetic cephalosporins, cephamycins, 1-oxacephems, clavulanic acids, penems, carbapenems, nocardicins, monobactams; tetracyclines, anhydrotetracyclines, anthracyclines; aminoglycosides; nucleosides such as N-nucleosides, C-nucleosides, carbocyclic nucleosides, blasticidin S; macrolides such as 12-membered ring macrolides, 14-membered ring macrolides, 16-membered ring macrolides; ansamycins; peptides such as bleomycins, gramicidins, polymyxins, bacitracins, large ring peptide antibiotics containing lactone linkages, actinomycins, amphomycin, capreomycin, distamycin, enduracidins, mikamycin, neocarzinostatin, stendomycin, viomycin, virginiamycin; cycloheximide; cycloserine; variotin; sarkomycin A; novobiocin; griseofulvin; chloramphenicol; mitomycins; fumagillin; monensins; pyrrolnitrin; fosfomycin; fusidic acid; D-(*p*-hydroxyphenyl)glycine; D-phenylglycine; enediynes.

Specific antibiotics which may be combined with the present compositions of the invention are for example benzylpenicillin (potassium, procaine, benzathine), phenoxymethylpenicillin (potassium), phenethicillin potassium, propicillin, carbenicillin (disodium, phenyl sodium, indanyl sodium), sulbenicillin, ticarcillin disodium, methicillin sodium, oxacillin sodium, cloxacillin sodium, dicloxacillin, flucloxacillin, ampicillin, mezlocillin, piperacillin sodium, amoxicillin, ciclacillin, hectacillin, sulbactam sodium, talampicillin hydrochloride, bacampicillin hydrochloride, pivmecillinam, cephalexin, cefaclor, cephaloglycin, cefadroxil, cephradine, cefroxadine, cephapirin sodium, cephalothin sodium, cephacetrile sodium, cefsulodin sodium, cephaloridine, cefatrizine, cefoperazone sodium, cefamandole, vefotiam hydrochloride, cefazolin sodium, ceftizoxime sodium, cefotaxime sodium, cefmenoxime hydrochloride, cefuroxime, ceftriaxone sodium, ceftazidime, cefoxitin, cefmetazole, cefotetan, latamoxef, clavulanic acid, imipenem, aztreonam, tetracycline, chlortetracycline hydrochloride, demethylchlortetracycline, oxytetracycline, methacycline, doxycycline, rolitetracycline, minocycline, daunorubicin hydrochloride, doxorubicin, aclarubicin, kanamycin sulfate, bekanamycin, tobramycin, gentamycin sulfate, dibekacin, amikacin, micronomicin, ribostamycin, neomycin sulfate, paromomycin sulfate, streptomycin sulfate, dihydrostreptomycin, destomycin A, hygromycin B, apramycin, sisomicin, netilmicin sulfate, spectinomycin hydrochloride, astromicin sulfate, validamycin, kasugamycin, polyoxin, blasticidin S, erythromycin, erythromycin estolate, oleandomycin phosphate, tracetyloleandomycin, kitasamycin, josamycin, spiramycin, tylosin, ivermectin, midecamycin, bleomycin sulfate, peplomycin sulfate, gramicidin S, polymyxin B, bacitracin, colistin sulfate, colistinmethanesulfonate sodium, enramycin, mikamycin, virginiamycin, capreomycin sulfate, viomycin, enviomycin, vancomycin, actinomycin D, neocarzinostatin, bestatin, pepstatin, monensin, lasalocid, salinomycin, amphotericin B, nystatin, natamycin, trichomycin, mithramycin, lincomycin, clindamycin, clindamycin palmitate hydrochloride, flavophospholipol, cycloserine, pecilocin, griseofulvin, chloramphenicol, chloramphenicol palmitate, mitomycin C, pyrrolnitrin, fosfomycin, fusidic acid, bicozamycin, tiamulin, siccanin.

Other Mycobacterial agents which may be combined with the compositions of the invention are for example rifampicin (=rifampin); isoniazid; pyrazinamide; amikacin; ethionamide; ethambutol; streptomycin; para-aminosalicylic acid; cycloserine; capreomycin; kanamycin; thioacetazone; PA-824; quinolones/fluoroquinolones such as for example moxifloxacin, gatifloxacin, ofloxacin, ciprofloxacin, sparfloxacin; macrolides such as for example clarithromycin, clofazimine, amoxycillin with clavulanic acid; rifamycins; rifabutin; rifapentine.

The term "about" as used herein in connection with a numerical value is meant to have its usual meaning in the context of the numerical value. Where necessary the word "about" may be replaced by the numerical value ±10%, or ±5%, or ±2%, or ±1%.

The following examples are intended to illustrate the present invention.

### Experimental part

The active ingredient, bedaquiline fumarate, may be prepared for example in accordance with the procedures described in international patent application WO 2008/068231. As seen from Table 1 below, TMC207 refers to Bedaquiline Fumarate.

### Examples

### 1) Effect of intra-granular filler

| **Ingredient** | | | |
|---|---|---|---|
| Intra-granular Part | | | |
| TMC207 | 24.18 | 24.18 | 24.18 |
| Silicified Microcrystalline cellulose | **14.91** | ...... | **29.82** |
| Mannitol | ...... | **29.82** | ...... |
| Glucose monohydrate | **14.91** | ...... | ...... |
| Crospovidone | 3.0 | 3.0 | 3.0 |
| Colloidal Silicon Dioxide | 2.0 | 2.0 | 2.0 |
| Hypromellose 5 cps | 3.0 | 3.0 | 3.0 |
| Polysorbate 20 | 0.2 | 0.2 | 0.2 |

| Extra-granular Part | | | |
|---|---|---|---|
| Silicified Microcrystalline cellulose | 32.3 | 32.3 | 32.3 |
| Crospovidone | 3.0 | 3.0 | 3.0 |
| Colloidal Silicon Dioxide | 0.5 | 0.5 | 0.5 |
| Sodium Stearyl Fumarate | 2.0 | 2.0 | 2.0 |
| Total (mg) | 100 | 100 | 100 |
| **Dispersion time*(sec)** | **30-70** | **40-70** | **40-50** |
| Hardness (N) | 20-31 | 24-31 | 25-40 |

The soluble excipient in the intra-granular part did not show any additional improvement in the dispersion time. It was observed that the time required for dispersion of tablet was adversely affected by addition of soluble excipients intragranularly. Thus, it was decided to continue with the Silicified MCC.

### 2) Effect of extra-granular fillers

| Ingredients | | |
|---|---|---|
| Intra-granular Part | | |
| TMC207 | 24.18 | 24.18 |
| Silicified Microcrystalline cellulose | 29.82 | 29.82 |
| Crospovidone | 3.0 | 3.0 |
| Colloidal Silicon Dioxide | 2.0 | 2.0 |
| Hypromellose 5 cps | 3.0 | 3.0 |
| Polysorbate 20 | 0.2 | 0.2 |

| Extra-granular Part | | |
|---|---|---|
| Mannitol | **32.3** | ...... |
| Silicified Microcrystalline cellulose | ...... | **32.3** |
| Crospovidone | 3.0 | 3.0 |
| Colloidal Silicon Dioxide | 0.5 | 0.5 |
| Sodium Stearyl Fumarate | 2.0 | 2.0 |
| Total | 100 | 100 |
| **Dispersion time*(sec)** | **60** | **45** |
| Hardness (N) | 19-25 | 20-27 |

The soluble excipient in the extra-granular part did not show any additional improvement. It was observed that the time required for dispersion of tablet was adversely affected by addition of soluble excipients. Thus, it was decided to continue with the Silicified MCC.

### 3) Effect of grade of Crospovidone

| Ingredients | | |
|---|---|---|
| Intra-granular Part | | |
| TMC207 | 24.18 | 24.18 |
| Silicified Microcrystalline cellulose | 29.82 | 29.82 |
| Crospovidone (Polyplasdone XL 10 ) | 3.0 | 3.0 |
| Colloidal Silicon Dioxide | 2.0 | 2.0 |
| Hypromellose 5 cps | 3.0 | 3.0 |
| Polysorbate 20 | 0.2 | 0.2 |

| Extra-granular Part | | |
|---|---|---|
| Silicified Microcrystalline cellulose | 32.3 | 32.3 |
| Crospovidone (Polyplasdone XL) | ...... | **3.0** |
| Crospovidone (Polyplasdone XL 10 ) | **3.0** | ...... |
| Colloidal Silicon Dioxide | 0.5 | 0.5 |
| Sodium Stearyl Fumarate | 2.0 | 2.0 |
| Total (mg) | 100 | 100 |
| **Dispersion time*(sec)** | **90-100** | **55-75** |
| Hardness (N) | 25-34 | 25-34 |

Crospovidone (Polyplasdone XL) **coarser grade** showed better dispersion pattern and behavior as compared to the fine particle Crospovidone (Polyplasdone XL 10), thus it was decided to further continue with the Polyplasdone XL grade intra-granularly as well as extra-granularly.

### Further Examples

The following composition of the invention was prepared in accordance with the teachniques described herein:

**Table 1: Current composition of TMC 207 dispersible tablet**

| **Ingredient** | **Function** | **Tablet quantity (mg)** | **Tablet quantity (%)** |
|---|---|---|---|
| BEDAQUILINE FUMARATE (TMC 207) | API | 24.18 | 24.18 |
| Silicified Microcrystalline Cellulose (Prosolv HD 90) | Filler | 29.82 | 29.82 |
| Crospovidone (Polyplasdone XL) | Disintegrant | 3.0 | 3.0 |
| Colloidal Silicon dioxide (Aerosil 200) | Glidant | 2.0 | 2.0 |
| Hypromellose 5 cps (Methocel E 5 LV) | Binder | 3.0 | 3.0 |
| Purified water ⁽¹⁾ | Solvent | qs | qs |
| Polysorbate 20 (Tween 20) | Surfactant | 0.2 | 0.2 |
| Silicified microcrystalline cellulose (Prosolv HD 90) | | 32.3 | 32.3 |
| Crospovidone (Polyplasdone XL) | Disintegrant | 3.0 | 3.0 |
| Colloidal Silicon dioxide (Aerosil 200) | Glidant | 0.5 | 0.5 |
| Sodium Stearyl Fumarate (Pruv) | Lubricant | 2.0 | 2.0 |
| **Tablet weight** | | 100 mg | 100% |

| | | | |
|---|---|---|---|
| (1) This material is a process aid and is removed during processing | | | |

### Stability data:

### Appearance, Assay and Chromatographic Purity

| Parameter | | Appearance | Water content of in % w/w | % Assay | Degradation compounds ≥0.05% (%) | | |
|---|---|---|---|---|---|---|---|
| | | | | Bedaquiline fumarate | | | |
| Test Method | | Visual Examination | KF- 00 131-V1 | HPLC-00129-V1 | | | |
| Storage Condition | Storage Time (Months) | Result | % w/w | Bedaquiline fumarate (%) | Any unspecified degradation product (%) U_RRT_0.74U_RRT_1.04 | | Total Degradation products (%) |
| | Initial | Pass | 4.4 | 98.1 | <0.05 | <0.05 | <0.05 |
| 25°C/60%RH | 1 | Pass | 5.2 | 98.7 | <0.05 | 0.05 | 0.05 |
| | 3 | Pass | 4.9 | 98.0 | <0.05 | <0.05 | <0.05 |
| | 6 | Pass | 4.6 | 99.1 | <0.05 | <0.05 | <0.05 |
| 30°C/75%RH | 1 | Pass | 5.0 | 99.2 | <0.05 | <0.05 | <0.05 |
| | 3 | Pass | 5.1 | 97.8 | <0.05 | <0.05 | <0.05 |
| | 6 | Pass | 5.3 | 99.0 | <0.05 | <0.05 | <0.05 |
| 40°C/75%RH | 1 | Pass | 4.8 | 99.1 | <0.05 | <0.05 | <0.05 |
| | 3 | Pass | 5.2 | 98.0 | <0.05 | 0.05 | 0.05 |
| | 6 | Pass | 5.4 | 99.1 | <0.05 | <0.05 | <0.05 |
| 50°C | 1 | Pass | 4.2 | 98.5 | <0.05 | <0.05 | <0.05 |
| | 3 | Pass | 4.1 | 98.2 | 0.08 | 0.06 | 0.14 |
| ICH Light | 8 hours | Pass | 5.3 | 98.4 | <0.05 | <0.05 | <0.05 |

Note: Light ICH: CIE85-ID65 700W/m² (Light study conducted on tablets packed in container) Where it is indicated "any specified degradation product", this may be an impurity in the API (for instance one that arises from the process).

The composition of the invention was found to be stable under ICH conditions.

### In Use Stability:

The stability for the dispersion of tablet in water was evaluated considering the lag time between the preparation and dosing of the dispersion.

### Method:-

As the composition of the invention (i.e. dispersible tablet described above) was stable for up to 6 hrs, it can be administered up to 6 hrs after its preparation.

5 tablet eq. to 100 mg dose were dispersed in 50 ml of water and further analyzed for assay and related substances after 0, 2, 4 and 6 hrs. in a clear glass flask at ambient conditions.

| **Sr. No.** | **Time** | **A known process impurity in %** | **Bedaquiline Fumarate in %** |
|---|---|---|---|
| 1 | 0 hrs. | 0.50 | 98.30 |
| 2 | 2 hrs. | 0.50 | 97.99 |
| 3 | 4 hrs. | 0.51 | 98.44 |
| 4 | 6 hrs. | 0.50 | 98.09 |

Product was found to be stable in the form of water dispersion for 6 hrs. Further it was stable in a clear glass contained too, medicating that it was not light-sensitive.

### Comparative dissolution profile of adult formulation vs Pediatric formulation via two different methods:

| **Formulation** | **Adult** | **Adult** | **Pediatric** | **Pediatric** |
|---|---|---|---|---|
| Batch No | 136695 | 136695 | HG-121019 | HG-121019 |
| Strength | 100mg | 100mg | 5 x 20mg | 5 x 20mg |
| Result set ID | 81938 | 82584 | 83398 | 83598 |
| Disso Media | 0.01N HCL | 0.01N HCL | 0.01N HCL | 0.01N HCL |
| No of units | 6 | 6 | 6 | 6 |
| Condition | RT_6M | RT_6M | RT_1.5Yrs | RT_1.5Yrs |
| Volume | 900 mL | 900 mL | 900 mL | 900 mL |
| Apparatus | Basket | Paddle | Basket | Paddle |
| RPM | 150 | 75 | 150 | 75 |
| 5 | 55.97 | 32.61 | 63.37 | 73.43 |
| 10 | 77.26 | 59.06 | 72.46 | 93.39 |
| 15 | 85.95 | 68.12 | 81.56 | 97.96 |
| 20 | 90.02 | 71.97 | 89.10 | 100.07 |
| 30 | 93.49 | 77.76 | 95.91 | 101.27 |
| 45 | 95.60 | 85.76 | 98.36 | 102.15 |
| 60 | 96.45 | 92.63 | 98.38 | 101.93 |
| 90 | 97.43 | 97.24 | 100.14 | 102.03 |
| F2 | | | 66.6** | 27.7* |

| | | | | |
|---|---|---|---|---|
| *wrt adult tablet; paddle 75 rpm **wrt adult tablet; basket 150 rpm RT = room temperature; 6M = 6 months F2: The FDA and EMEA define similarity factor as a "logarithmic reciprocal square root transformation of one plus the mean squared (the average sum of squares) differences of drug percent dissolved between the test and the reference products". If F2 >50 the products are similar and if less than 50 then dissimilar. | | | | |

Dissolution profile of pediatric formulation was faster as compared to adult formulation in both the dissolution methods.

### Effect on Bioavailability:

1) Food effect on bioavailability was reduced as compared to adult tablet

| | Regular breakfast | Yoghurt |
|---|---|---|
| Adult tablet | 91% | 32% |
| Water dispersible tablet | 82% | 17% |

2) Both adult and paediatric formulation tested with regular breakfast and yogurt showed that both formulations are bioequivalent despite dispersible tablets showing faster dissolution profile in vitro.

### Panel 1 (standard breakfast)

| Water dispersible tablet | **Ratio (%)** | **90% CI** |
|---|---|---|
| Cmax | 106.58 | 96.11 - 118.18 |
| AUC₇₂ₕ | 98.43 | 91.85 - 105.47 |

### Panel 2 (yoghurt)

| Water dispersible tablet | **Ratio (%)** | **90% CI** |
|---|---|---|
| Cmax | 111.93 | 104.26 - 120.16 |
| AUC₇₂ₕ | 112.95 | 105.94 - 120.42 |

Hence, the composition of the invention was found to be bioequivalent to adult conventional tablet formulation when tested in adult population in fasted, fed and with yogurt. The product (invention) decreased the food effect by 9% and by 15% when dosed with yogurt as compared to adult tablet formulation.

### Additional Examples

Further development in the formulation was aimed at catering the clinical need of 10 mg dose increments. This was accomplished with developing a 20 mg formulation with a breakline to enable dosing in 10 mg dose increments if needed.

To achieve this, following changes were carried out in previous formulation that was tested in the bioequivalence study.

| ***Parameter*** | ***Bedquiline fumarate Dispersible tablet 20 mg (Formulation described above)*** | ***Bedquiline fumarate Dispersible tablet 20 mg (Further formulation)*** |
|---|---|---|
| Weight | 100 mg | 200 mg |
| Tablet shape | Circular | Caplet |
| Tablet surface | Plain on both sides | Break line on both sides |
| Tablet Debossing | None | "2" & "0" separated by break line on one side and plain with break line or other side |
| Hardness | 20-40 N | 70-120N |
| Dose(covered) | 20 mg | 10 mg and 20 mg |
| Dispersion time | About 30 sec (in 50 ml) | About 60 sec (in 50ml) - can be dispersed in 5 ml water |
| Percentage of MCC (Avicel PH 102)- Insoluble excipient/diluent | 62.12% | 73.71% |
| Percentage of API | 24.18% | 12.09% |

### Composition of further formulation:

| ***Ingredients*** | ***Bedaquiline fumarate Dispersible tablet 20 mg (Formulation of 100 mg weight described above)*** | | ***Bedaquiline fumarate Dispersible tablet 20 mg (Further formulation of 200 mg weight)*** | |
|---|---|---|---|---|
| | mg/unit | Percent w/w | mg/unit | Percent w/w |

| **Intra Granular Portion** | | | | |
|---|---|---|---|---|
| Bedaquiline fumarate | 24.18 | 24.18 | 24.18 | 12.09 |
| Silicified Microcrystalline Cellulose | 29.32 | 29.32 | 82.82 | 41.41 |
| Crospovidone | 3.00 | 3 | 6 | 3 |
| Colloidal Silicon Dioxide | 2.0 | 2 | 4 | 2 |
| Hypromellose 5 cps | 3.5 | 3.5 | 7 | 3.5 |
| Polysorbate 20 | 0.2 | 0.2 | 0.4 | 0.2 |
| Total (Intragranular Portion) | 62.2 | 62.2 | 124.4 | 62.2 |

| **Extra granular Portion** | | | | |
|---|---|---|---|---|
| Sodium Stearyl Fumarate | 2.0 | 2 | 4 | 2 |
| Silicified Microcrystalline | 32.3 | 32.3 | 64.6 | 32.3 |
| Crospovidone | 3.0 | 3 | 6 | 3 |
| Colloidal Silicon Dioxide | 0.5 | 0.5 | 1 | 0.5 |
| Total (Extragranular Portion) | 37.8 | 37.8 | 75.6 | 37.8 |
| Tablet weight (mg) | **100.0** | **100** | **200** | **100** |

### Formulation trials to study various factors in formulation

### 1) Effect of Intragranular filler Vs. Extragranular filler

| **Ingredients** | Example 1 | Example 2 |
|---|---|---|
| Intra-granular Portion | | |
| Bedaquiline fumarate | 24.18 | 24.18 |
| Silicified Microcrystalline Cellulose (Prosolv SMCC HD90) | 82.82 | 115.12 |
| Crospovidone (Polyplasdone XL) | 6.00 | 6.00 |
| Colloidal Silicon Dioxide (Aerosil 200Pharma) | 4.00 | 4.00 |
| Hypromellose 5 cps (Methocel E5 LV) | 7.00 | 7.00 |
| Polysorbate 20 (Tween 20 HP) | 0.40 | 0.40 |
| Purified Water* | - | - |
| Weight of Intragranular | 124.4 | 156.7 |

| Extra-granular Portion | | |
|---|---|---|
| Sodium Stearyl Fumarate (Pruv) | 4.00 | 4.00 |
| Silicified Microcrystalline Cellulose (Prosolv SMCC HD90) | 64.60 | 32.30 |
| Crospovidone (Polyplasdone XL) | 6.00 | 6.00 |
| Colloidal Silicon Dioxide (Aerosil 200 Pharma) | 1.00 | 1.00 |
| Dispersion time | 75 sec (5 ml) | 70-75 sec (5 ml) |
| | 50-55 sec (50 ml) | |
| Hardness | 93-100 N (95N) | 92-107 N (96N) |
| Tablet weight (mg) | 200.00 | 200.00 |

| | | |
|---|---|---|
| **Conclusion:** - Intragranular filler (silicified MCC) when varied between 41% to 58% did not show any impact on the critical quality attributes (CQAs) of the product. This change directly impacts the extragranular filler concentration which when varied between 32% and 16% does not impact the CQA's. | | |

### Effect of amount of Crospovidone

| **Ingredients** | Example 1 | Example 2 |
|---|---|---|
| Intra-granular Portion | | |
| Bedaquiline fumarate | 24.18 | 24.18 |
| Silicified Microcrystalline Cellulose (Prosolv SMCC HD90) | 82.82 | 85.82 |
| Crospovidone (Polyplasdone XL) | 6.00 | 3.00 |
| Colloidal Silicon Dioxide (Aerosil 200Pharma) | 4.00 | 4.00 |
| Hypromellose 5 cps (Methocel E5 LV) | 7.00 | 7.00 |
| Polysorbate 20 (Tween 20 HP) | 0.40 | 0.40 |
| Purified Water* | - | - |
| Weight of Intragranular | 124.4 | 124.4 |

| Extra-granular Portion | | |
|---|---|---|
| Sodium Stearyl Fumarate (Pruv) | 4.00 | 4.00 |
| Silicified Microcrystalline Cellulose (Prosolv SMCC HD90) | 64.60 | 67.60 |
| Crospovidone (Polyplasdone XL) | 6.00 | 3.00 |
| Colloidal Silicon Dioxide (Aerosil 200 Pharma) | 1.00 | 1.00 |
| Dispersion time | 75 sec (5 ml) | 85-90 sec (5 ml) |
| | 50-55 sec (50 ml) | |
| Hardness | 93-100 N (95N) | 87-105 N (95N) |
| Tablet weight (mg) | 200.00 | 200.00 |

| | | |
|---|---|---|
| **Conclusion:** - Disintegrant (Crospovidone) concentrations were studied between 3% to 6% ranges in the formulation. This does not impact the CQA's but the dispersion time with 6% Crospovidone is slightly better than with 3% | | |

### 2) Effect of amount of increased amount of binder

| **Ingredients** | Example 1 | Example 2 |
|---|---|---|
| Intra-granular Portion | | |
| Bedaquiline fumarate | 24.18 | 24.18 |
| Silicified Microcrystalline Cellulose (Prosolv SMCC HD90) | 82.82 | 79.32 |
| Crospovidone (Polyplasdone XL) | 6.00 | 6.00 |
| Colloidal Silicon Dioxide (Aerosil 200Pharma) | 4.00 | 4.00 |
| Hypromellose 5 cps (Methocel E5 LV) | 7.00 | 10.50 |
| Polysorbate 20 (Tween 20 HP) | 0.40 | 0.40 |
| Purified Water* | - | - |
| Weight of Intragranular | 124.4 | 124.4 |

| Extra-granular Portion | | |
|---|---|---|
| Sodium Stearyl Fumarate (Pruv) | 4.00 | 4.00 |
| Silicified Microcrystalline Cellulose (Prosolv SMCC HD90) | 64.60 | 64.60 |
| Crospovidone (Polyplasdone XL) | 6.00 | 6.00 |
| Colloidal Silicon Dioxide (Aerosil 200 Pharma) | 1.00 | 1.00 |
| Dispersion time | 75 sec (5 ml) | 135-145 sec (5 ml) |
| | 50-55 sec (50 ml) | |
| Hardness | 93-100 N (95N) | 89-104 N (95N) |
| Tablet weight (mg) | 200.00 | 200.00 |

| | | |
|---|---|---|
| **Conclusion:-** Binder ( HPMC) concentration was studied between 3.5% and 5.25%. Higher cone of binder showed higher dispersion time. | | |

### Initial & Stability data of further dispersible tablet formulation:

| **Further Formulation (of 200 mg tablet weight)** | **Assay (%)** | **RS (%)** | **Water by KF (%)** |
|---|---|---|---|
| Initial sample | 99.45 | <RT | 5.0 |
| HDPE Bottle 1M 40°C/75 % RH | 99.74 | <RT | 4.1 |
| HDPE Bottle 2M 40°C/75 % RH | 98.30 | <RT | 4.0 |
| HDPE Bottle 3M 40°C/75 % RH | 99.30 | 0.05 | 3.1 |
| HDPE Bottle 6M 40°C/75 % RH | 100.6 | 0.15 | 3.2 |

| | | | |
|---|---|---|---|
| RT: Reporting threshold Formulation exhibited satisfactory stability till 6 months in HDPE Bottles. | | | |

### Dissolution profile (0.01N HCl) - Comparison of 100mg dispersible tablet formulation vs 200mg dispersible tablet formulation

| **Time in minutes** | **100mg tablet formulation** | **200mg tablet formulation** |
|---|---|---|
| | % Release | |
| 5 | 80 | 78 |
| 10 | 98 | 93 |
| 15 | 101 | 95 |
| 20 | 101 | 96 |
| 30 | 102 | 97 |
| 45 | 103 | 97 |

| | | |
|---|---|---|
| 100mg & 200mg tablet formulation dissolution profile is comparable. | | |

### Split tablet data:

### 1 - Weight loss of split tablets

| **Intact weight of tablet (mg)** | **Fraction-1** | **Fraction-2** | **Weight loss (%)** |
|---|---|---|---|
| 202.9 | 100.8 | 102.2 | -0.05 |
| 203.6 | 100.4 | 103 | 0.10 |
| 203 | 105.3 | 97.8 | -0.05 |
| 201.5 | 103.4 | 97.3 | 0.40 |
| 201.5 | 102.6 | 99 | -0.05 |
| 202.1 | 103.4 | 98.7 | 0.00 |
| 201.6 | 102.2 | 99.3 | 0.05 |
| 202.8 | 103.3 | 99.4 | 0.05 |
| 202.1 | 102.7 | 99.3 | 0.05 |
| 203.1 | 101.8 | 101.1 | 0.10 |
| 202.1 | 101.6 | 100.5 | 0.00 |
| 203.8 | 105 | 98.9 | -0.05 |
| 201 | 101.2 | 99.5 | 0.15 |
| 202 | 101.5 | 100.3 | 0.10 |
| 202.6 | 103.1 | 99.4 | 0.05 |
| 202.9 | 100.8 | 102.2 | -0.05 |
| SD (for weight of split tablets, n=30): 2.04 | | | |
| RSD (for weight of split tablets n=30): 2.02 % | | | |

| | | | |
|---|---|---|---|
| Friability of split tablets (100 revolutions): 0.06 % Friability of split tablets (400 revolutions): 0.16 % | | | |

## Claims

1. A tablet comprising bedaquiline fumarate as the active ingredient and wherein the tablet comprises an intra-granular and extra-granular layer in which:
the intra-granular layer comprises a non-soluble excipient/diluent and is charaterised in that the intra-granular layer is absent a soluble excipient/diluent that is starch; and
- the extra-granular layer comprises a non-soluble excipient/diluent.

2. A tablet as claimed in Claim 1, wherein the intra-granular layer is absent any soluble excipient/diluent.

3. A tablet as claimed in Claim 1 or Claim 2, wherein the non-soluble excipient/diluent in the intra-granular layer is microcrystalline cellulose.

4. A tablet comprising bedaquiline fumarate as the active ingredient and wherein the tablet comprises an intra-granular and extra-granular layer in which the intra-granular layer comprises a non-soluble excipient/diluent that is microcrystalline cellulose and the extra-granular layer comprises a non-soluble excipient/diluent that is microcrystalline cellulose.

5. A tablet as claimed in Claim 4, wherein the intra-granular layer is absent a soluble excipient/diluent.

6. A tablet composition wherein the different parts of the composition, specifically the intra-granular and extra-granular fraction and binder portion, comprise the following ingredients by weight based on the total weight of the composition:
Intra-granular fraction
5 to 50% of active ingredient bedaquiline fumarate
10 to 50% of non-soluble excipient/diluent
1 to 5% disintegrant
0.1 to 5% glidant
Binder
1 to 10% binder or polymer
0.01 to 5% wetting agent or surfactant
Solvent (qs), e.g. water - if necessary (i.e. only the amount needed, if any)
Extra-granular fraction
10 to 50% of a non-soluble excipient/diluent
1 to 5% disintegrant
0 to 3% glidant
0 to 5% lubricant

7. A tablet composition as claimed in Claim 6, wherein the intra-granular layer is absent any soluble excipient/diluent; and/or, optionally, the extra-granular fraction is also absent any soluble excipient/diluent.

8. A tablet composition as claimed in Claim 6 or Claim 7, which consists of the following compositions of intra-granular fraction, binder and extra-granular fraction, by weight based on the total weight of the composition:
Intra-granular fraction
24.18% (or about 25%) of active ingredient bedaquiline fumarate
29.82% (or about 30%) of non-soluble excipient/diluent
3% (or about 3%) disintegrant
2 % (or about 2%) glidant
Binder
3% (or about 3%) binder or polymer
0.2% (or about 0.2%) wetting agent or surfactant
Solvent (qs), e.g. water - if necessary (i.e. only the amount needed, if any)
Extra-granular fraction
32.3% (or about 30%) of excipient/diluent
3% (or about 3%) disintegrant
0.5 % (or about 0.5%) glidant
2% (or about 2%) lubricant

9. A tablet composition as claimed in Claim 6 or Claim 7, which consists of the following compositions of intra-granular fraction, binder and extra-granular fraction, by weight based on the total weight of the composition:
Intra-granular fraction
12.09% of active ingredient bedauiline fumarate
41.41% of non-soluble excipient/diluent (silicified microcrystalline cellulose)
3% disintegrant (crospovidone)
2 % glidant (colloidal silicon dioxide)
Binder
3.5% binder or polymer (hypromellose 5 cps)
0.2% wetting agent or surfactant (polysorbate 20)
Solvent (qs), e.g. water - if necessary (i.e. only the amount needed, if any)
Extra-granular fraction
32.3% of excipient/diluent (silicified microcrystalline cellulose)
3% disintegrant (crospovidone)
0.5 % glidant (colloidal silicon dioxide)
2% lubricant (sodium stearyl fumarate)

10. A tablet composition as claimed in claim 8, which consists of the following:
Intra-granular fraction
24.18 mg bedaquiline fumarate
29.82 mg silicified microcrystalline cellulose
3 mg crospovidone
2 mg colloidal silicon dioxide
3 mg hypromellose 5 cps
0.2 mg polysorbate 20
Purified water qs
Extra-granular fraction
32.3 mg silicified microcrystalline cellulose
3 mg crospovidone
0.5 mg colloidal silicon dioxide
2 mg sodium stearyl fumarate

11. A tablet composition as claimed in claim 9, which consists of the following:
Intra-granular fraction
24.18 mg bedaquiline fumarate
82.82 mg silicified microcrystalline cellulose
6 mg crospovidone
4 mg colloidal silicon dioxide
7 mg hypromellose 5 cps
0.4 mg polysorbate 20
Extra-granular fraction
64.6 mg silicified microcrystalline cellulose
6 mg crospovidone
1 mg colloidal silicon dioxide
4 mg sodium stearyl fumarate

12. A tablet composition wherein the different parts of the composition, specifically the intra-granular and extra-granular fraction and binder portion, comprise the following ingredients by weight based on the total weight of the composition:
Intra-granular fraction
10 to 30% of active ingredient bedaquiline fumarate
20 to 40% of non-soluble excipient/diluent microcrystalline cellulose
2 to 4% disintegrant crospovidone
0.5 to 4% glidant colloidal silicon dioxide
Binder
2 to 5% binder or polymer hypromellose 5 cps
0.1 to 1% wetting agent or surfactant polysorbate 20
Solvent (qs), e.g. water - if necessary (i.e. only the amount needed, if any)
Extra-granular fraction
20 to 40% of excipient/diluent microcrystalline cellulose
2 to 4% disintegrant crospovidone
0 to 1% glidant colloidal silicon dioxide
1 to 3% lubricant sodium stearyl fumarate

13. A tablet or tablet composition as claimed in any one of the preceding claims for use in the treatment of tuberculosis.

14. A tablet or tablet composition for use according to Claim 13, for use in the pediatric and/or geriatric population.

15. A tablet or tablet composition as claimed in any one of the preceding claims for use in the treatment of tuberculosis in combination with one or more other therapeutic agent(s) useful in the treatment of tuberculosis.

16. A combination for use in the treatment of tuberculosis comprising a tablet or tablet composition as claimed in any one of Claims 1 to 14 and one or more other therapeutic agents useful in the treatment of tuberculosis.

17. A process for preparing a tablet composition as claimed in any one of Claims 1 to 12 which comprises:
(a) obtaining an intra-granular fraction using the intra-granular fraction components mentioned in any one of Claim 6 to 12;
(b) preparing a binder fraction, using the binder fraction components mentioned in any one of Claims 6 to 12;
(c) obtaining an extra-granular fraction using the extra-granular fraction components mentioned in any one of Claims 6 to 12,
and using those fractions to prepare a composition.

18. A tablet composition obtainable by the process as defined in Claim 17.

## Patentansprüche

1. Tablette, umfassend Bedaquilinfumarat als den Wirkstoff, wobei die Tablette eine intragranuläre und eine extragranuläre Schicht umfasst, wobei:
die intragranuläre Schicht einen unlöslichen Exzipienten/ein unlösliches Verdünnungsmittel umfasst und **dadurch gekennzeichnet ist, dass** in der intragranulären Schicht kein löslicher Exzipient/kein lösliches Verdünnungsmittel, der/das Stärke ist, vorhanden ist; und
die extragranuläre Schicht einen unlöslichen Exzipienten/ein unlösliches Verdünnungsmittel umfasst.

2. Tablette gemäß Anspruch 1, wobei in der intragranulären Schicht kein löslicher Exzipient/kein lösliches Verdünnungsmittel vorhanden ist.

3. Tablette gemäß Anspruch 1 oder Anspruch 2, wobei der unlösliche Exzipienten/das unlösliche Verdünnungsmittel in der intragranulären Schicht mikrokristalline Cellulose ist.

4. Tablette, umfassend Bedaquilinfumarat als den Wirkstoff, wobei die Tablette eine intragranuläre und eine extragranuläre Schicht umfasst, wobei die intragranuläre Schicht einen unlöslichen Exzipienten/ein unlösliches Verdünnungsmittel, der/das mikrokristalline Cellulose ist, umfasst, und die extragranuläre Schicht einen unlöslichen Exzipienten/ein unlösliches Verdünnungsmittel, der/das mikrokristalline Cellulose ist, umfasst.

5. Tablette gemäß Anspruch 4, wobei in der intragranulären Schicht kein löslicher Exzipient/kein lösliches Verdünnungsmittel vorhanden ist.

6. Tablettenzusammensetzung, wobei die verschiedenen Teile der Zusammensetzung, insbesondere die intragranuläre und die extragranuläre Fraktion und der Bindemittelanteil, die folgenden Inhaltsstoffe nach Gewicht, bezogen auf das Gesamtgewicht der Zusammensetzung, umfassen:
Intragranuläre Fraktion
5 bis 50 % Wirkstoff Bedaquilinfumarat
10 bis 50 % unlöslicher Exzipient/unlösliches Verdünnungsmittel
1 bis 5 % Sprengmittel
0,1 bis 5 % Gleitmittel
Bindemittel
1 bis 10 % Bindemittel oder Polymer
0,01 bis 5 % Netzmittel oder Tensid
Lösungsmittel (qs), z. B. Wasser - falls erforderlich (d.h. nur die benötigte Menge, wenn überhaupt)
Extragranuläre Fraktion
10 bis 50 % unlöslicher Exzipient/unlösliches Verdünnungsmittel
1 bis 5 % Sprengmittel
0 bis 3 % Gleitmittel
0 bis 5 % Schmiermittel.

7. Tablettenzusammensetzung gemäß Anspruch 6, wobei in der intragranulären Schicht kein löslicher Exzipient/kein lösliches Verdünnungsmittel vorhanden ist; und/oder gegebenenfalls in der extragranulären Fraktion ebenfalls kein löslicher Exzipient/kein lösliches Verdünnungsmittel vorhanden ist.

8. Tablettenzusammensetzung gemäß Anspruch 6 oder Anspruch 7, die aus den folgenden Zusammensetzungen von intragranulärer Fraktion, Bindemittel und extragranulärer Fraktion nach Gewicht, bezogen auf das Gesamtgewicht der Zusammensetzung, besteht:
Intragranuläre Fraktion
24,18 % (oder etwa 25 %) Wirkstoff Bedaquilinfumarat
29,82 % (oder etwa 30 %) unlöslicher Exzipient/unlösliches Verdünnungsmittel
3 % (oder etwa 3 %) Sprengmittel
2 % (oder etwa 2 %) Gleitmittel
Bindemittel
3 % (oder etwa 3 %) Bindemittel oder Polymer
0,2 % (oder etwa 0,2 %) Netzmittel oder Tensid
Lösungsmittel (qs), z. B. Wasser - falls erforderlich (d.h. nur die benötigte Menge, wenn überhaupt)
Extragranuläre Fraktion
32,3 % (oder etwa 30 %) unlöslicher Exzipient/unlösliches Verdünnungsmittel
3 % (oder etwa 3 %) Sprengmittel
0,5 % (oder etwa 0,5 %) Gleitmittel
2 % (oder etwa 2 %) Schmiermittel.

9. Tablettenzusammensetzung gemäß Anspruch 6 oder Anspruch 7, die aus den folgenden Zusammensetzungen von intragranulärer Fraktion, Bindemittel und extragranulärer Fraktion nach Gewicht, bezogen auf das Gesamtgewicht der Zusammensetzung, besteht:
Intragranuläre Fraktion
12,09 % Wirkstoff Bedaquilinfumarat
41,41 % unlöslicher Exzipient/unlösliches Verdünnungsmittel (silifizierte mikrokristalline Cellulose)
3 % Sprengmittel (Crospovidon)
2 % Gleitmittel (kolloidales Siliciumdioxid)
Bindemittel
3,5 % Bindemittel oder Polymer (Hypromellose 5 cps)
0,2 % Netzmittel oder Tensid (Polysorbat 20)
Lösungsmittel (qs), z. B. Wasser - falls erforderlich (d.h. nur die benötigte Menge, wenn überhaupt)
Extragranuläre Fraktion
32,3 % Exzipient/Verdünnungsmittel (silifizierte mikrokristalline Cellulose)
3 % Sprengmittel (Crospovidon)
0,5 % Gleitmittel (kolloidales Siliciumdioxid)
2 % Schmiermittel (Natriumstearylfumarat).

10. Tablettenzusammensetzung gemäß Anspruch 8, die aus folgendem besteht:
Intragranuläre Fraktion
24,18 mg Bedaquilinfumarat
29,82 mg silifizierte mikrokristalline Cellulose
3 mg Crospovidon
2 mg kolloidales Siliciumdioxid
3 mg Hypromellose 5 cps
0,2 mg Polysorbat 20
Gereinigtes Wasser qs
Extragranuläre Fraktion
32,3 mg silifizierte mikrokristalline Cellulose
3 mg Crospovidon
0,5 mg kolloidales Siliciumdioxid
2 mg Natriumstearylfumarat.

11. Tablettenzusammensetzung gemäß Anspruch 9, die aus folgendem besteht:
Intragranuläre Fraktion
24,18 mg Bedaquilinfumarat
82,82 mg silifizierte mikrokristalline Cellulose
6 mg Crospovidon
4 mg kolloidales Siliciumdioxid
7 mg Hypromellose 5 cps
0,4 mg Polysorbat 20
Extragranuläre Fraktion
64,6 mg silifizierte mikrokristalline Cellulose
6 mg Crospovidon
1 mg kolloidales Siliciumdioxid
4 mg Natriumstearylfumarat.

12. Tablettenzusammensetzung, wobei die verschiedenen Teile der Zusammensetzung, insbesondere die intragranuläre und die extragranuläre Fraktion und der Bindemittelanteil, die folgenden Inhaltsstoffe nach Gewicht, bezogen auf das Gesamtgewicht der Zusammensetzung, umfassen:
Intragranuläre Fraktion
10 bis 30% Wirkstoff Bedaquilinfumarat
20 bis 40 % unlöslicher Exzipient/unlösliches Verdünnungsmittel mikrokristalline Cellulose
2 bis 4 % Sprengmittel Crospovidon
0,5 bis 4 % Gleitmittel kolloidales Siliciumdioxid
Bindemittel
2 bis 5 % Bindemittel oder Polymer Hypromellose 5 cps
0,1 bis 1 % Netzmittel oder Tensid Polysorbat 20
Lösungsmittel (qs), z. B. Wasser - falls erforderlich (d.h. nur die benötigte Menge, wenn überhaupt)
Extragranuläre Fraktion
20 bis 40 % Exzipient/Verdünnungsmittel mikrokristalline Cellulose
2 bis 4 % Sprengmittel Crospovidon
0 bis 1 % Gleitmittel kolloidales Siliciumdioxid
1 bis 3 % Schmiermittel Natriumstearylfumarat.

13. Tablette oder Tablettenzusammensetzung gemäß einem der vorstehenden Ansprüche zur Verwendung bei der Behandlung von Tuberkulose.

14. Tablette oder Tablettenzusammensetzung zur Verwendung gemäß Anspruch 13 zur Verwendung bei der pädiatrischen und/oder geriatrischen Bevölkerung.

15. Tablette oder Tablettenzusammensetzung gemäß einem der vorstehenden Ansprüche zur Verwendung bei der Behandlung von Tuberkulose in Kombination mit einem oder mehreren weiteren Therapeutika, die zur Behandlung von Tuberkulose geeignet sind.

16. Kombination zur Verwendung bei der Behandlung von Tuberkulose, umfassend eine Tablette oder eine Tablettenzusammensetzung gemäß einem der Ansprüche 1 bis 14 und ein oder mehrere weitere Therapeutika, die zur Behandlung von Tuberkulose geeignet sind.

17. Verfahren zur Herstellung einer Tablettenzusammensetzung gemäß einem der Ansprüche 1 bis 12, umfassend:
(a) Erhalten einer intragranulären Fraktion unter Verwendung der in einem der Ansprüche 6 bis 12 genannten Komponenten der intragranulären Fraktion;
(b) Herstellen einer Bindemittelfraktion unter Verwendung der in einem der Ansprüche 6 bis 12 genannten Komponenten der Bindemittelfraktion;
(c) Erhalten einer extragranulären Fraktion unter Verwendung der in einem der Ansprüche 6 bis 12 genannten Komponenten der extragranulären Fraktion,
und Verwenden dieser Fraktionen zur Herstellung einer Zusammensetzung.

18. Tablettenzusammensetzung, erhältlich durch das Verfahren gemäß Anspruch 17.

## Revendications

1. Comprimé comprenant du fumarate de bédaquiline en tant qu'ingrédient actif et le comprimé comprenant une couche intra-granulaire et extra-granulaire dans lequel :
la couche intra-granulaire comprend un excipient/diluant non soluble et est **caractérisée en ce que** la couche intra-granulaire ne comprend pas un excipient/diluant soluble qui est un amidon ; et
- la couche extra-granulaire comprend un excipient/diluant non soluble.

2. Comprimé selon la revendication 1, la couche intra-granulaire ne comprenant aucun excipient/diluant soluble.

3. Comprimé selon la revendication 1 ou la revendication 2, l'excipient/le diluant non soluble dans la couche intra-granulaire étant une cellulose microcristalline.

4. Comprimé comprenant du fumarate de bédaquiline en tant qu'ingrédient actif et le comprimé comprenant une couche intra-granulaire et extra-granulaire dans lequel la couche intra-granulaire comprend un excipient/diluant non soluble qui est une cellulose microcristalline et la couche extra-granulaire comprend un excipient/diluant non soluble qui est une cellulose microcristalline.

5. Comprimé selon la revendication 4, la couche intra-granulaire ne comprenant pas un excipient/diluant soluble.

6. Composition de comprimé, les différentes parties de la composition, spécifiquement la fraction intra-granulaire et extra-granulaire et la partie de liant, comprenant les ingrédients suivants en poids sur la base du poids total de la composition :
fraction intra-granulaire
5 à 50 % d'ingrédient actif fumarate de bédaquiline
10 à 50 % d'un excipient/diluant non soluble
1 à 5 % d'un désintégrant
0,1 à 5 % d'un agent glissant
liant
1 à 10 % d'un liant ou polymère
0,01 à 5 % d'un agent mouillant ou d'un tensioactif
solvant (qs), par exemple de l'eau - si nécessaire (c'est-à-dire seulement la quantité nécessaire, s'il y a lieu)
fraction extra-granulaire
10 à 50 % d'un excipient/diluant non soluble
1 à 5 % d'un désintégrant
0 à 3 % d'un agent glissant
0 à 5 % d'un lubrifiant.

7. Composition de comprimé selon la revendication 6, la couche intra-granulaire ne comprenant aucun excipient/diluant soluble ; et/ou, éventuellement, la fraction extra-granulaire ne comprenant également aucun excipient/diluant soluble.

8. Composition de comprimé selon la revendication 6 ou la revendication 7, qui est constituée des compositions suivantes de fraction intra-granulaire, liant et fraction extra-granulaire, en poids sur la base du poids total de la composition :
fraction intra-granulaire
24,18 % (ou environ 25 %) d'ingrédient actif fumarate de bédaquiline
29,82 % (ou environ 30 %) d'excipient/de diluant non soluble
3 % (ou environ 3 %) de désintégrant
2 % (ou environ 2 %) d'agent glissant
liant
3 % (ou environ 3 %) de liant ou de polymère
0,2 % (ou environ 0,2 %) d'agent mouillant ou de tensioactif
solvant (qs), par exemple de l'eau - si nécessaire (c'est-à-dire seulement la quantité nécessaire, s'il y a lieu)
fraction extra-granulaire
32,3 % (ou environ 30 %) d'excipient/de diluant
3 % (ou environ 3 %) de désintégrant
0,5 % (ou environ 0,5 %) d'agent glissant
2 % (ou environ 2 %) de lubrifiant.

9. Composition de comprimé selon la revendication 6 ou la revendication 7, qui est constituée des compositions suivantes de fraction intra-granulaire, liant et fraction extra-granulaire, en poids sur la base du poids total de la composition :
fraction intra-granulaire
12,09 % d'ingrédient actif fumarate de bédaquiline
41,41 % d'excipient/de diluant non soluble (cellulose microcristalline silicifiée)
3 % de désintégrant (crospovidone)
2 % d'agent glissant (dioxyde de silicium colloïdal)
liant
3,5 % de liant ou de polymère (hypromellose 5 cps)
0,2 % d'agent mouillant ou de tensioactif (polysorbate 20)
solvant (qs), par exemple de l'eau - si nécessaire (c'est-à-dire seulement la quantité nécessaire, s'il y a lieu)
fraction extra-granulaire
32,3 % d'excipient/de diluent (cellulose microcristalline silicifiée)
3 % de désintégrant (crospovidone)
0,5 % d'agent glissant (dioxyde de silicium colloïdal)
2 % de lubrifiant (stéarylfumarate de sodium).

10. Composition de comprimé selon la revendication 8, qui est constituée de ce qui suit :
fraction intra-granulaire
24,18 mg de fumarate de bédaquiline
29,82 mg de cellulose microcristalline silicifiée
3 mg de crospovidone
2 mg de dioxyde de silicium colloïdal
3 mg d'hypromellose 5 cps
0,2 mg de polysorbate 20
de l'eau purifiée qs
fraction extra-granulaire
32,3 mg de cellulose microcristalline silicifiée
3 mg de crospovidone
0,5 mg de dioxyde de silicium colloïdal
2 mg de stéarylfumarate de sodium.

11. Composition de comprimé selon la revendication 9, qui est constituée de ce qui suit :
fraction intra-granulaire
24,18 mg de fumarate de bédaquiline
82,82 mg de cellulose microcristalline silicifiée
6 mg de crospovidone
4 mg de dioxyde de silicium colloïdal
7 mg d'hypromellose 5 cps
0,4 mg de polysorbate 20
fraction extra-granulaire
64,6 mg de cellulose microcristalline silicifiée
6 mg de crospovidone
1 mg de dioxyde de silicium colloïdal
4 mg de stéarylfumarate de sodium.

12. Composition de comprimé, les différentes parties de la composition, spécifiquement la fraction intra-granulaire et extra-granulaire et la partie de liant, comprenant les ingrédients suivants en poids sur la base du poids total de la composition :
fraction intra-granulaire
10 à 30 % d'ingrédient actif fumarate de bédaquiline
20 à 40 % d'excipient/de diluant non soluble cellulose microcristalline
2 à 4 % de désintégrant crospovidone
0,5 à 4 % d'agent glissant dioxyde de silicium colloïdal
liant
2 à 5 % d'un liant ou polymère hypromellose 5 cps
0,1 à 1 % d'agent mouillant ou tensioactif polysorbate 20
solvant (qs), par exemple de l'eau - si nécessaire (c'est-à-dire seulement la quantité nécessaire, s'il y a lieu)
fraction extra-granulaire
20 à 40 % d'excipient/de diluant cellulose microcristalline
2 à 4 % de désintégrant crospovidone
0 à 1 % d'agent glissant dioxyde de silicium colloïdal
1 à 3 % de lubrifiant stéarylfumarate de sodium.

13. Comprimé ou composition de comprimé selon l'une quelconque des revendications précédentes pour une utilisation dans le traitement de la tuberculose.

14. Comprimé ou composition de comprimé pour une utilisation selon la revendication 13, pour une utilisation dans la population pédiatrique et/ou gériatrique.

15. Comprimé ou composition de comprimé selon l'une quelconque des revendications précédentes pour une utilisation dans le traitement de la tuberculose en combinaison avec un ou plusieurs autres agents thérapeutiques utiles dans le traitement de la tuberculose.

16. Combinaison pour une utilisation dans le traitement de la tuberculose comprenant un comprimé ou une composition de comprimé selon l'une quelconque des revendications 1 à 14 et un ou plusieurs autres agents thérapeutiques utiles dans le traitement de la tuberculose.

17. Procédé pour la préparation d'une composition de comprimé selon l'une quelconque des revendications 1 à 12 qui comprend :
(a) l'obtention d'une fraction intra-granulaire, en utilisant les composants de fraction intra-granulaire mentionnés dans l'une quelconque des revendications 6 à 12 ;
(b) la préparation d'une fraction de liant, en utilisant les composants de fraction de liant mentionnés dans l'une quelconque des revendications 6 à 12 ;
(c) l'obtention d'une fraction extra-granulaire, en utilisant les composants de fraction extra-granulaire mentionnés dans l'une quelconque des revendications 6 à 12,
et l'utilisation de ces fractions pour préparer une composition.

18. Composition de comprimé pouvant être obtenue par le procédé tel que défini dans la revendication 17.
